(19) Europäisches Patentamt
European Patent Office
Office européen des brevets

(11) Publication number: **0 306 323 B1**

(12) # EUROPEAN PATENT SPECIFICATION

(45) Date of publication of patent specification: **21.09.94**

(51) Int. Cl.⁵: **C07D 471/04**, C07D 487/04, A61K 31/435, A61K 31/55, //(C07D471/04,221:00,209:00), (C07D487/04,223:00,209:00)

(21) Application number: **88308135.8**

(22) Date of filing: **02.09.88**

(54) **Lactam derivatives.**

(30) Priority: **03.09.87 GB 8720695**
**15.08.88 GB 8819382**

(43) Date of publication of application:
**08.03.89 Bulletin 89/10**

(45) Publication of the grant of the patent:
**21.09.94 Bulletin 94/38**

(84) Designated Contracting States:
**AT BE CH DE ES FR GB GR IT LI LU NL SE**

(56) References cited:
**EP-A- 0 191 562**

(73) Proprietor: **GLAXO GROUP LIMITED**
**Clarges House**
**6-12 Clarges Street**
**London W1Y 8DH (GB)**

(72) Inventor: **Coates, Ian Harold**
**20, Mandeville Road**
**Hertford Hertfordshire (GB)**
Inventor: **North, Peter Charles**
**60, Downlands**
**Royston Hertfordshire, SG8 5BY (GB)**
Inventor: **Oxford, Alexander William**
**60, Green Drift**
**Royston Hertfordshire (GB)**

(74) Representative: **Marchant, James Ian et al**
**Elkington and Fife**
**Prospect House**
**8 Pembroke Road**
**Sevenoaks, Kent TN13 1XR (GB)**

EP 0 306 323 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to lactam derivatives, to processes for their preparation, to pharmaceutical compositions containing them and to their medical use.

In particular the invention relates to compounds which are potent and selective antagonists of 5-hydroxytryptamine (5-HT) at 5-HT receptors of the type located on terminals of primary afferent nerves. Receptors of this type are now designated as 5-$HT_3$ receptors and are also present in the central nervous system. 5-HT occurs widely in the neuronal pathways in the central nervous system and disturbance of these 5-HT containing pathways is known to alter behavioural syndromes such as mood, psychomotor activity, appetite and memory.

Compounds having antagonist activity at 5-$HT_3$ receptors have been described previously.

Thus for example published UK Patent Specification GB-A-2153821 and published European Patent Specifications EP-A-191562, 219193 and 210840 disclose 3-imidazolylmethyltetrahydrocarbazolones which may be represented by the general formula:

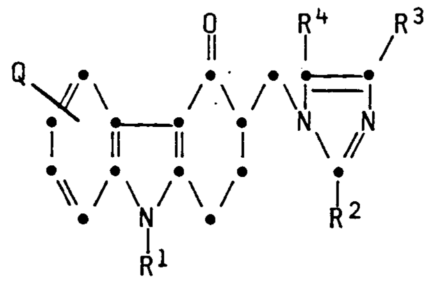

wherein $R^1$ represents a hydrogen atom or a group selected from $C_{1-10}$alkyl, $C_{3-6}$ alkenyl, $C_{3-10}$ alkynyl, $C_{3-7}$ cycloalkyl, $C_{3-7}$cycloalkyl$C_{1-4}$alkyl, phenyl or phenyl$C_{1-3}$alkyl, and in the case where Q represents a hydrogen atom, $R^1$ may also represent $-CO_2R^5$,$-COR^5$, $-CONR^5R^6$- or $-SO_2R^5$ (wherein $R^5$ and $R^6$, which may be the same or different, each represents a hydrogen atom, a $C_{1-6}$ alkyl or $C_{3-7}$cycloalkyl group, or a phenyl or phenyl$C_{1-4}$alkyl group, in which the phenyl group is optionally substituted by one or more $C_{1-4}$ alkyl, $C_{1-4}$alkoxy or hydroxy groups or halogen atoms, with the proviso that $R^5$ does not represent a hydrogen atom when $R^1$ represents a group $-CO_2R^5$ or $-SO_2R^5$);

one of the groups represented by $R^2$, $R^3$ and $R^4$ is a hydrogen atom or a $C_{1-6}$ alkyl, $C_{3-7}$ cycloalkyl, $C_{2-6}$ alkenyl, or phenyl$C_{1-3}$alkyl group, and each of the other two groups, which may be the same or different, represents a hydrogen atom or a $C_{1-6}$ alkyl group;

Q represents a hydrogen atom or a halogen atom or a hydroxy, $C_{1-4}$alkoxy, phenyl$C_{1-3}$alkoxy or $C_{1-6}$ alkyl group or a group $-NR^7R^8$ or $-CONR^7R^8$ (wherein $R^7$ and $R^8$, which may be the same or different, each represents a hydrogen atom or a $C_{1-4}$ alkyl or $C_{3-4}$ alkenyl group, or together with the nitrogen atom to which they are attached form a saturated 5 to 7 membered ring);

and physiologically acceptable salts and solvates thereof.

We have now found a novel group of compounds which differ in structure from those described previously, and which are potent antagonists of the effect of 5-HT at 5-$HT_3$ receptors.

The present invention provides a tricyclic lactam of the general formula (I):

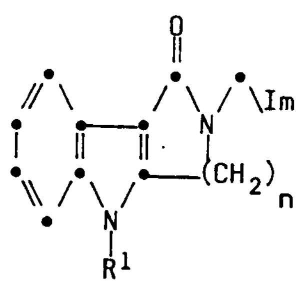

(I)

wherein Im represents an imidazolyl group of the formula :

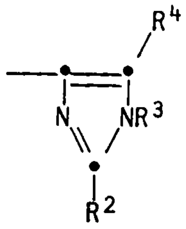

or

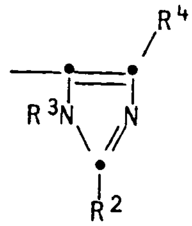

and $R^1$ represents a hydrogen atom or a group selected from $C_{1-6}$alkyl, $C_{3-6}$alkenyl, $C_{3-10}$alkynyl, $C_{3-7}$cycloalkyl, $C_{3-7}$cycloalkyl$C_{1-4}$ alkyl, phenyl, phenyl$C_{1-3}$alkyl, phenylmethoxymethyl, phenoxyethyl, phenoxymethyl, $-CO_2R^5$, $-COR^5$, $-CONR^5R^6$ or $-SO_2R^5$ (wherein $R^5$ and $R^6$, which may be the same or different, each represents a hydrogen atom, a $C_{1-6}$ alkyl or $C_{3-7}$ cycloalkyl group, or a phenyl or phenyl$C_{1-4}$alkyl group, in which the phenyl group is optionally substituted by one or more $C_{1-4}$ alkyl, $C_{1-4}$ alkoxy or hydroxy groups or halogen atoms, with the proviso that $R^5$ does not represent a hydrogen atom when $R^1$ represents a group $-CO_2R^5$ or $-SO_2R^5$);

one of the groups represented by $R^2$, $R^3$ and $R^4$ is a hydrogen atom or a $C_{1-6}$alkyl, $C_{3-7}$cycloalkyl, $C_{3-6}$alkenyl, phenyl or phenyl$C_{1-3}$alkyl group, and each of the other two groups, which may be the same or different, represents a hydrogen atom or a $C_{1-6}$ alkyl group;

n represents 2 or 3;

and physiologically acceptable salts and solvates thereof.

According to one aspect, the invention provides compounds of formula (I) wherein $R^1$ represents a hydrogen atom or a group selected from $C_{1-6}$ alkyl, $C_{3-6}$ alkenyl, $C_{3-10}$ alkynyl, $C_{3-7}$cycloalkyl, $C_{3-7}$cycloalkyl$C_{1-4}$ alkyl, phenyl or phenyl$C_{1-3}$ alkyl (n and Im being as defined in formula (I)).

Suitable physiologically acceptable salts of the compounds of general formula (I) include acid addition salts formed with organic or inorganic acids for example, hydrochlorides, hydrobromides, sulphates, alkyl- or arylsulphonates (e.g. methanesulphonates or p-toluenesulphonates), phosphates, acetates, citrates, succinates, tartrates, fumarates and maleates. The solvates may, for example, be hydrates.

All optical isomers of compounds of general formula (I) and their mixtures including the racemic mixtures thereof, and all the geometric isomers of compounds of formula (I), are embraced by the invention.

Referring to the general formula (I), an alkyl group may be a straight chain or branched chain alkyl group, for example, methyl, ethyl, n-propyl, prop-2-yl, n-butyl, but-2-yl, 2-methylprop-2-yl, n-pentyl, pent-3-yl or n-hexyl. A $C_{3-6}$ alkenyl group may be, for example, a propenyl or butenyl group. When $R^1$ represents a $C_{3-6}$alkenyl or $C_{3-10}$alkynyl group, or $R^3$ represents a $C_{3-6}$alkenyl group, or $R^7$ or $R^8$ represents a $C_{3-4}$alkenyl group, the double or triple bond may not be adjacent to the nitrogen atom. A phenyl$C_{1-3}$alkyl group may be, for example, a benzyl, phenethyl or 3-phenylpropyl group. A $C_{3-7}$cycloalkyl group may be, for example, a cyclopropyl, cyclobutyl, cyclopentyl, cyclohexyl or cycloheptyl group.

A preferred class of compounds of formula (I) is that wherein $R^1$ represents a hydrogen atom or a $C_{1-6}$ alkyl (e.g. methyl, ethyl, n-propyl, prop-2-yl), $C_{3-4}$ alkenyl (e.g. prop-2-enyl), $C_{3-4}$ alkynyl (e.g. prop-2-ynyl), $C_{5-6}$cycloalkyl (e.g. cyclopentyl), $C_{5-6}$cycloalkylmethyl (e.g. cyclopentylmethyl), phenyl$C_{1-2}$ alkyl (e.g. benzyl), phenylmethoxymethyl, N,N-di$C_{1-3}$alkylcarboxamido (e.g. N,N-dimethylcarboxamido) or $C_{1-3}$alkylsulphonyl (e.g. methylsulphonyl) group. More preferably $R^1$ represents a $C_{1-4}$ alkyl (e.g. methyl or n-propyl), $C_{3-4}$alkynyl (e.g. prop-2-ynyl), $C_{5-6}$cycloalkyl (e.g. cyclopentyl), $C_{5-6}$cycloalkylmethyl (e.g. cyclopentylmethyl), phenyl$C_{1-2}$ alkyl (e.g. benzyl), phenylmethoxymethyl, or N,N-di$C_{1-3}$alkylcarboxamido (e.g. N,N-dimethylcarboxamido) group.

Another preferred class of compounds of formula (I) is that wherein $R^2$ represents a hydrogen atom or a $C_{1-3}$ alkyl (e.g. methyl) group, more preferably a hydrogen atom.

Another preferred class of compounds of formula (I) is that wherein $R^3$ represents a hydrogen atom or a $C_{1-3}$ alkyl (e.g. methyl) group, more preferably a hydrogen atom.

A further preferred class of compounds of formula (I) is that wherein $R^4$ represents a hydrogen atom or a $C_{1-3}$ alkyl (e.g. methyl or n-propyl) group. Most preferably $R^4$ represents a methyl group.

When $R^2$ and $R^3$ represent hydrogen atoms, $R^4$ is preferably $C_{1-6}$alkyl, $C_{3-7}$ cycloalkyl, $C_{3-6}$ alkenyl or phenyl$C_{1-3}$alkyl, more particularly $C_{1-6}$ alkyl.

A further preferred class of compounds of formula (I) is that wherein n represents 2.

A preferred group of compounds of formula (I) is that wherein $R^1$ represents a hydrogen atom or a $C_{1-4}$ alkyl, $C_{3-4}$alkenyl, $C_{3-4}$alkynyl, $C_{5-6}$cycloalkyl, $C_{5-6}$cycloalkylmethyl, phenyl$C_{1-2}$ alkyl, phenylmethox-

ymethyl, N,N-di$C_{1-3}$alkylcarboxamido or $C_{1-3}$alkylsulphonyl group; $R^2$ represents a hydrogen atom; and $R^3$ and $R^4$ each represent a hydrogen atom or a $C_{1-3}$ alkyl group.

A particularly preferred group of compounds of formula (I) is that wherein $R^1$ represents a methyl, n-propyl, prop-2-ynyl, cyclopentyl, cyclopentylmethyl, benzyl or N, N-dimethylcarboxamido group; $R^2$ and $R^3$ each represent a hydrogen atom; and $R^4$ represents a methyl group.

Within the above preferred and particularly preferred groups of compounds, an especially important group of compounds is that in which n represents 2.

Preferred compounds according to the invention are:
2,3,4,5-tetrahydro-5-methyl-2-[(5-methyl-1H-imidazol-4-yl)methyl]-1H-pyrido[4,3-b]indol-1-one;
2,3,4,5-tetrahydro-5-(phenylmethyl)-2-[(5-methyl-1H-imidazol-4-yl)methyl]-1H-pyrido[4,3-b]indol-1-one;
5-cyclopentyl-2,3,4,5-tetrahydro-2-[(5-methyl-1H-imidazol-4-yl)methyl]-1H-pyrido[4,3-b]indol-1-one;
2,3,4,5-tetrahydro-2-[(5-methyl-1H-imidazol-4-yl)methyl]-5-propyl-1H-pyrido[4,3-b]indol-1-one;
5-(cyclopentylmethyl)-2,3,4,5-tetrahydro-2-[(5-methyl-1H-imidazol-4-yl)methyl]-1H-pyrido[4,3-b]indol-1-one;
3,4,5,6-tetrahydro-6-methyl-2-[(5-methyl-1H-imidazol-4-yl)methyl]azepino[4,3-b]indol-1(2H)-one;
2,3,4,5-tetrahydro-N,N-dimethyl-2-[(5-methyl-1H-imidazol-4-yl)methyl]-1-oxo-5H-pyrido[4,3-b]indole-5-carboxamide;
2,3,4,5-tetrahydro-2-[(5-methyl-1H-imidazol-4-yl)methyl]-5-(2-propynyl)-1H-pyrido[4,3-b]indol-1-one;
and their physiologically acceptable salts and solvates.

The potent and selective antagonism of 5-HT at $5\text{-}HT_3$ receptors by compounds of the invention has been demonstrated by their ability to inhibit 3-(5-methyl-1H-imidazol-4-yl)-1-[1-(methyl-$t_3$)-1H-indol-3-yl]-1-propanone binding in rat entorhinal cortex homogenates (following the general procedure described by G. Kilpatrick et al. in Nature, 1987, 330, 746), and/or by their ability to inhibit the 5-HT-induced depolarisation of the rat isolated vagus nerve preparation.

In addition to their activity as potent and selective antagonists of 5-HT at $5\text{-}HT_3$ receptors, certain compounds according to the invention have the advantage of an extended duration of action.

A particularly preferred compound on account of both its potency and duration of action is 2,3,4,5-tetrahydro-5-methyl-2-[(5-methyl-1H-imidazol-4-yl)methyl]-1H-pyrido[4,3-b]indol-1-one and its physiologically acceptable salts and solvates. Preferred salts of this compound are the hydrochloride and maleate.

Compounds of formula (I), which antagonise the effect of 5-HT at $5\text{-}HT_3$ receptors, are useful in the treatment of conditions such as psychotic disorders (e.g. schizophrenia and mania); anxiety; and nausea and vomiting, particularly that associated with cancer chemotherapy and radiotherapy. Compounds of formula (I) are also useful in the treatment of gastric stasis; symptoms of gastrointestinal dysfunction such as occur with dyspepsia, peptic ulcer, reflux oesophagitis, flatulence and irritable bowel syndrome; migraine; and pain. Compounds of formula (I) may also be used in the treatment of dependency on drugs and substances of abuse, depression, and dementia and other cognitive disorders.

According to another aspect, the invention provides a method of treatment of a human or animal subject suffering from a psychotic disorder such as schizophrenia or mania; or from anxiety; nausea or vomiting; gastric stasis; symptoms of gastrointestinal dysfunction such as dyspepsia, reflux oesophagitis, peptic ulcer, flatulence and irritable bowel syndrome; migraine; or pain, which comprises administering an effective amount of a compound of formula (I) or a physiologically acceptable salt or solvate thereof.

Accordingly, the invention also provides a pharmaceutical composition which comprises at least one compound selected from compounds of the general formula (I), and their physiologically acceptable salts and solvates (e.g. hydrates), for use in human or veterinary medicine, and formulated for administration by any convenient route.

Such compositions may be formulated in conventional manner using one or more physiologically acceptable carriers and/or excipients.

Thus the compounds according to the invention may be formulated for oral, buccal, parenteral or rectal administration or in a form suitable for administration by inhalation or insufflation (either through the mouth or nose).

For oral administration, the pharmaceutical compositions may take the form of, for example, tablets or capsules prepared by conventional means with pharmaceutically acceptable excipients such as binding agents (e.g. pregelatinised maize starch, polyvinylpyrrolidone or hydroxylpropyl methylcellulose); fillers (e.g. lactose, microcrystalline cellulose or calcium hydrogen phosphate); lubricants (e.g. magnesium stearate, talc or silica); disintegrants (e.g. potato starch or sodium starch glycollate); or wetting agents (e.g. sodium lauryl sulphate). The tablets may be coated by methods well known in the art. Liquid preparations for oral administration may take the form of, for example, solutions, syrups or suspensions, or they may be presented as a dry product for constitution with water or other suitable vehicle before use. Such liquid preparations may be prepared by conventional means with pharmaceutically acceptable additives such as

suspending agents (e.g. sorbitol syrup, cellulose derivatives or hydrogenated edible fats); emulsifying agents (e.g. lecithin or acacia); non-aqueous vehicles (e.g. almond oil, oily esters, ethyl alcohol or fractionated vegetable oils); and preservatives (e.g. methyl or propyl-p-hydroxybenzoates or sorbic acid). The preparations may also contain buffer salts, flavouring, colouring and sweetening agents as appropriate.

Preparations for oral administration may be suitably formulated to give controlled release of the active compound.

For buccal administration the compositions may take the form of tablets or lozenges formulated in conventional manner.

The compounds of the invention may be formulated for parenteral administration by bolus injection or continuous infusion. Formulations for injection may be presented in unit dosage form e.g. in ampoules or in multi-dose containers, with an added preservative. The compositions may take such forms as suspensions, solutions or emulsions in oily or aqueous vehicles, and may contain formulatory agents such as suspending, stabilising and/or dispersing agents. Alternatively, the active ingredient may be in powder form for constitution with a suitable vehicle, e.g. sterile pyrogen-free water, before use.

The compounds of the invention may also be formulated in rectal compositions such as suppositories or retention enemas, e.g. containing conventional suppository bases such as cocoa butter or other glycerides.

In addition to the formulations described previously, the compounds of the invention may also be formulated as depot preparations. Such long acting formulations may be administered by implantation (for example subcutaneously or intramuscularly) or by intramuscular injection. Thus, for example, the compounds of the invention may be formulated with suitable polymeric or hydrophobic materials (for example as an emulsion in an acceptable oil) or ion exchange resins, or as sparingly soluble derivatives, for example, as a sparingly soluble salt.

For administration by inhalation the compounds according to the invention are conveniently delivered in the form of an aerosol spray presentation from pressurised packs or a nebuliser, with the use of a suitable propellant, e.g. dichlorodifluoromethane, trichlorofluoromethane, dichlorotetrafluoroethane, carbon dioxide or other suitable gas. In the case of a pressurised aerosol the dosage unit may be determined by providing a valve to deliver a metered amount. Capsules and cartridges of e.g. gelatin for use in an inhaler or insufflator may be formulated containing a powder mix of a compound of the invention and a suitable powder base such as lactose or starch.

For intranasal administration, the compounds according to the invention may be formulated as solutions for administration via a suitable metered or unit dose device or alternatively as a powder mix with a suitable carrier for administration using a suitable delivery device.

The compounds of formula (I) may also be administered in combination with other therapeutic agents. Thus, for example, in the treatment of gastric stasis, symptoms of gastrointestinal dysfunction and nausea and vomiting, the compounds of formula (I) may be administered in combination with antisecretory agents such as histamine $H_2$-receptor antagonists (e.g. ranitidine, sufotidine, 1-methyl-5-[[3-[3-(1-piperidinylmethyl)-phenoxy]propyl]amino]-1H-1,2,4-triazole-3-methanol, cimetidine, famotidine, nizatidine or roxatidine) or $H^+K^+$ATPase inhibitors (e.g. omeprazole).

A proposed dose of the compounds of the invention for administration to man (of approximately 70kg body weight) is 0.001 to 100mg, preferably 0.01 to 50mg, more preferably 0.1 to 20mg of the active ingredient per unit dose expressed as the weight of free base, which could be administered, for example, 1 to 4 times per day. It will be appreciated that it may be necessary to make routine variations to the dosage, depending on the age and conditions of the patient. The dosage will also depend on the route of administration.

Compounds of general formula (I) and physiologically acceptable salts or solvates thereof may be prepared by the general methods outlined hereinafter. In the following description, the groups $R^1$, n, and Im are as defined for compounds of general formula (I) unless otherwise stated.

According to a first general process (A), a compound of general formula (I) may be prepared by alkylating a compound of formula (II):

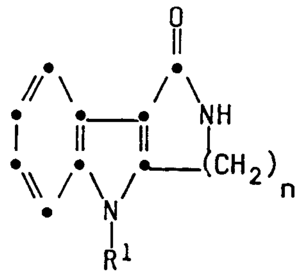

(II)

with a compound of formula (III):

$LCH_2$-Im (III)

or a protected derivative thereof, wherein L represents a leaving atom or group, such as a halogen atom (e.g. chlorine, bromine or iodine), or an acyloxy group (e.g. trifluoroacetyloxy or acetoxy), or a sulphonyloxy group (e.g. trifluoromethanesulphonyloxy, p-toluenesulphonyloxy or methanesulphonyloxy), followed where necessary by removal of any protecting groups. L is preferably a halogen atom (e.g. a chlorine atom).

The reaction may be carried out in an inert solvent such as an ether (e.g. dimethoxyethane, diglyme or tetrahydrofuran), a substituted amide (e.g. dimethylformamide or N-methylpyrrolidone), an aromatic hydrocarbon (e.g. toluene), a ketone (e.g. acetone), or dimethyl sulphoxide, at a temperature between ambient and 100 °C, in the presence of a base. Suitable bases include alkali metal hydrides (e.g. sodium hydride), alkali metal carbonates (e.g. sodium carbonate), alkali metal amides (e.g. sodium amide), alkali metal alkoxides (e.g. potassium t-butoxide) or alkali metal hydroxides (e.g. sodium or potassium hydroxide).

According to another general process (B), a compound of general formula (I) wherein n represents 2, may be prepared by hydrogenation of a compound of formula (IV):

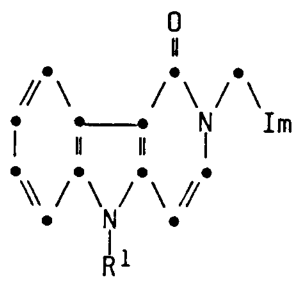

(IV)

or a protected derivative thereof, followed where necessary by removal of any protecting groups.

Hydrogenation according to general process (B) may be effected using conventional procedures, for example using hydrogen in the presence of a noble metal catalyst (e.g. palladium, Raney nickel, platinum or rhodium). The catalyst may be supported on, for example, charcoal or alumina, or alternatively a homogeneous catalyst such as tris(triphenylphosphine)rhodium chloride may be used. The hydrogenation will generally be effected in a solvent such as an alcohol (e.g. methanol or ethanol), an ether (e.g. dioxan), or an ester (e.g. ethyl acetate), or in a mixture of an alcohol and either a hydrocarbon (e.g. toluene) or a halogenated hydrocarbon (e.g. dichloromethane), at a temperature in the range -20 to +100 °C, and at a pressure of from 1 to 10 atmospheres.

According to another general process (C), a compound of general formula (I) may be prepared by cyclising a compound of formula (V):

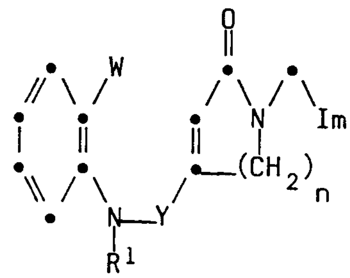

(V)

wherein W represents a hydrogen atom and Y represents the group NH, or W represents a halogen atom and Y represents a bond, or a salt or protected derivative thereof, followed where necessary by removal of any protecting groups.

According to one embodiment (a) of process (C), the reaction is effected with a compound of formula (V) wherein W represents a hydrogen atom and Y represents the group NH, and the cyclisation may be carried out in aqueous or non-aqueous media, in the presence of an acid catalyst.

It will be appreciated that these compounds of formula (V) may exist in the corresponding enol hydrazone tautomeric form.

When an aqueous medium is employed this may be water or a mixture of water and a organic solvent such as an alcohol (e.g. methanol, ethanol or isopropanol) or an ether (e.g. dioxan or tetrahydrofuran). The acid catalyst may be, for example, an inorganic acid such as concentrated hydrochloric or sulphuric acid. In some cases the acid catalyst may also act as the reaction solvent. In an anhydrous reaction medium, which may comprise one or more alcohols or ethers (e.g. as described above), carboxylic acids (e.g. acetic acid) or esters (e.g. ethyl acetate), the acid catalyst may alternatively be a Lewis acid such as boron trifluoride, zinc chloride or magnesium chloride. The cyclisation reaction may conveniently be carried out at temperatures of from 20 to 200 °C, preferably 20 to 125 °C.

Alternatively the cyclisation according to embodiment (a) of process (C) may be carried out in the presence of polyphosphate ester in a reaction medium which may comprise one or more organic solvents, preferably halogenated hydrocarbons such as chloroform, dichloromethane, dichloroethane, dichlorodifluoromethane, or mixtures thereof. Polyphosphate ester is a mixture of esters which may be prepared from phosphorus pentoxide, diethyl ether and chloroform according to the method described in 'Reagents for Organic Synthesis', (Fieser and Fieser, John Wiley and Sons, 1967).

According to another embodiment (b) of process (C), the reaction is effected with a compound of formula (V) wherein W represents a halogen atom, for example, a chlorine atom or, more preferably, a bromine or iodine atom, Y represents a bond, and the cyclisation is effected photochemically.

The reaction may conveniently be effected by irradiating with a mercury lamp, preferably a medium or high pressure mercury lamp. Suitable solvents include nitriles (e.g. acetonitrile), chlorinated hydrocarbons (e.g. carbon tetrachloride) and cyclic ethers (e.g. tetrahydrofuran or dioxan) and mixtures thereof. The reaction may conveniently be effected in the presence of a base such as a tertiary amine (.e.g. triethylamine).

According to another general process (D), a compound of general formula (I) wherein $R^3$ represents a hydrogen atom, may be prepared by the reaction of a compound of formula (VI):

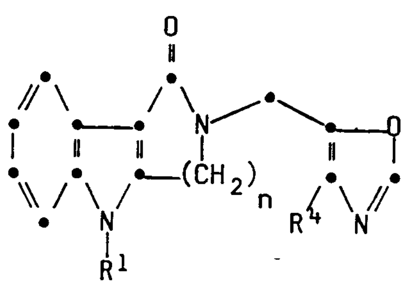

(VI)

or a protected derivative thereof, with formamide, at a temperature in the range of 150 to 200 °C, followed where necessary by removal of any protecting groups.

According to another general process (E), a compound of general formula (I) may be prepared by reacting a compound of formula (VII):

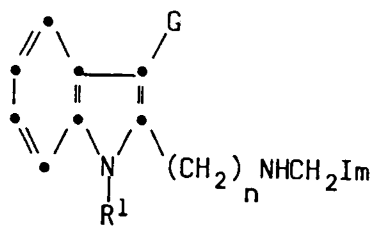

(VII)

wherein G represents a hydrogen atom, or a protected derivative thereof, with phosgene in the presence of a Lewis acid; or by reacting a compound of formula (VII) wherein G represents an iodine or a bromine atom, or a protected derivative thereof, with carbon monoxide in the presence of a palladium (II) salt, followed where necessary by removal of any protecting groups.

According to one embodiment of process (E), a compound of formula (VII), wherein G represents a hydrogen atom, is reacted with phosgene in the presence of a Lewis acid such as anhydrous aluminium trichloride or stannic chloride. The reaction may conveniently be effected in an inert solvent such as an aromatic hydrocarbon (e.g. toluene) or a halogenated hydrocarbon (e.g. dichloromethane), or mixtures thereof, and at a temperature between ambient and 100 °C.

According to another embodiment of process (E), a compound of formula (VII), wherein G represents an iodine or a bromine atom, is reacted with carbon monoxide in the presence of a palladium (II) salt (e.g. palladium acetate or palladium chloride) and preferably in the presence of triphenylphosphine. The reaction may conveniently be effected in a solvent such as a tertiary amine (e.g. tri-n-butylamine), optionally in the presence of a co-solvent such as an ether (e.g. tetrahydrofuran) or an aromatic hydrocarbon (e.g. toluene), at a temperature in the range 100 to 150 °C, and at atmospheric pressure.

According to another general process (F), a compound of general formula (I) may be converted into another compound of formula (I) using conventional techniques. Such conventional techniques include hydrogenation, alkylation and acylation using protection and deprotection where necessary.

Thus, according to one embodiment of the interconversion process (F), hydrogenation may be used to convert an alkenyl or an alkynyl substituent into an alkyl substituent, or an alkynyl into an alkenyl substituent. Hydrogenation may also be used to replace a phenylmethoxymethyl group by a hydrogen atom. Hydrogenation according to general process (F) may be effected using conventional procedures, for example, using hydrogen in the presence of a catalyst, as described above in general process (B).

The term 'alkylation' according to general process (F) includes the introduction of groups such as cycloalkyl, alkenyl or phenalkyl groups.

Thus, for example, a compound of formula (I) in which $R^1$ represents a $C_{1-6}$ alkyl, $C_{3-6}$ alkenyl, $C_{3-10}$ alkynyl, $C_{3-7}$ cycloalkyl, $C_{3-7}$cycloalkyl$C_{1-4}$ alkyl, phenyl $C_{1-3}$ alkyl, phenylmethoxymethyl, phenoxyethyl or phenoxymethyl group may be prepared by alkylating a compound of formula (I) in which $R^1$ represents a hydrogen atom, or a compound in which $R^3$ represents a $C_{1-6}$alkyl, $C_{3-7}$cycloalkyl, $C_{3-6}$alkenyl or phenyl$C_{1-3}$alkyl group may be prepared by alkylating the corresponding compound of formula (I) in which $R^3$ represents a hydrogen atom, using conventional procedures, for example as described in published European Patent Specification EP-A-242973. Thus the reactions may be effected using an appropriate alkylating agent of formula $R^7Z$ (where $R^7$ is the group to be introduced and Z is a leaving atom or group), preferably in the presence of a base.

According to another embodiment of general process (F), a compound of formula (I) wherein $R^1$ represents $-CO_2R^5$, $-COR^5$, $-CONR^5R^6$ or $-SO_2R^5$ may be prepared by acylating or sulphonylating as appropriate, a compound of formula (I) wherein $R^1$ represents a hydrogen atom. The acylation/sulphonylation reactions may be effected using an appropriate acylating/sulphonylating agent according to conventional procedures, for example, as described in published European Patent Specification No. 210840.

It should be appreciated that in the above transformations it may be necessary or desirable to protect any sensitive groups in the molecule of the compound in question to avoid undesirable side reactions. For example, it may be necessary to protect the indole and/or imidazole nitrogen atoms, for example with an arylmethyl (e.g. trityl), arylmethoxymethyl (e.g. phenylmethoxymethyl), alkyl (e.g. t-butyl), alkoxymethyl (e.g. methoxymethyl), acyl (e.g. benzyloxycarbonyl) or a sulphonyl (e.g. N,N-dimethylaminosulphonyl or p-toluenesulphonyl) group.

Thus according to another general process (G), a compound of general formula (I) may be prepared by the removal of any protecting groups from a protected form of a compound of formula (I). Deprotection may be effected using conventional techniques such as those described in 'Protective Groups in Organic

Synthesis' by T. W. Greene (John Wiley and Sons, 1981).

For example, an arylmethoxymethyl N-protecting group may be cleaved by hydrogenolysis in the presence of a catalyst (e.g. palladium on charcoal). A trityl group may be cleaved by acid hydrolysis (e.g. using dilute hydrochloric or acetic acid). An alkoxyalkyl group may be removed using a mineral acid (e.g. dilute hydrochloric or hydrobromic acid). An acyl group may be removed by hydrolysis under acidic or basic conditions (e.g. using hydrogen bromide, dilute hydrochloric acid or sodium hydroxide). A sulphonyl group may also be removed by alkaline or acidic hydrolysis, and an N, N-dimethylaminosulphonyl group may also be removed (e.g. from an imidazole nitrogen atom) by photolysis.

Compounds of formula (II) may be obtained by a Beckmann rearrangement of an oxime of formula (VIII):

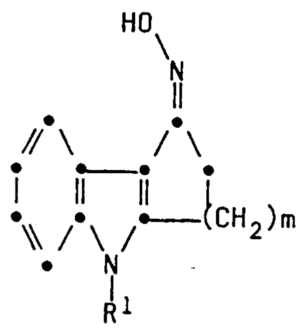

(VIII)

wherein m represents 1 or 2, or a protected derivative thereof. The Beckmann rearrangement may be effected using conventional methods, for example by using an acid (e.g. polyphosphoric or sulphuric acid, or a mixture of hydrochloric acid, acetic anhydride and acetic acid) in an inert solvent such as an ether (e.g. dioxan), an amide (e.g. dimethylformamide) or a hydrocarbon (e.g. toluene or cyclohexane), at an elevated temperature of, for example, 50 to 120 °C. Alternatively, the hydroxy group of the oxime of formula (VIII), may be converted into a leaving group such as a chloride (using, for example, phosphorus pentachloride) or a hydrocarbylsulphonate (e.g. a mesylate or a tosylate) or a trifluoroacetate group (using conventional acylation methods). Subsequent heating at a temperature of, for example, 20 to 150 °C, in an inert solvent as described above, gives a compound of formula (II).

Compounds of formula (VIII) may be prepared from the corresponding tricyclic ketone of formula (IX):

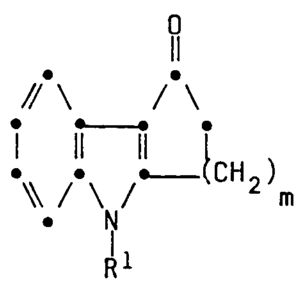

(IX)

wherein m represents 1 or 2, or a protected derivative thereof using conventional methods, for example by using hydroxylamine hydrochloride in a solvent such as pyridine.

Compounds of formula (IV) may be prepared, for example, by reacting a compound of formula (X):

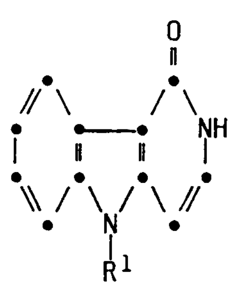

(X)

or a protected derivative thereof, with a compound of formula (III) wherein L is as defined previously, or a protected derivative thereof, using the conditions described in process (A).

Compounds of formula (X) may be prepared by heating a compound of formula (II) wherein n represents 2, with a noble metal catalyst such as palladium, palladium oxide, platinum or nickel, at a temperature of, for example, 300 to 350 °C. The catalyst may be supported on, for example, charcoal or alumina, and the reaction may optionally be carried out in the presence of an inert solvent such as an aromatic hydrocarbon (e.g. p-cymene)

Compounds of formula (V) wherein W represents a hydrogen atom and Y represents the group NH may be prepared by the reaction of a compound of formula (XI):

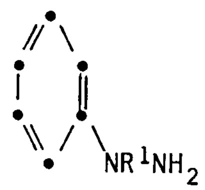

(XI)

or a salt thereof, with a compound of formula (XII):

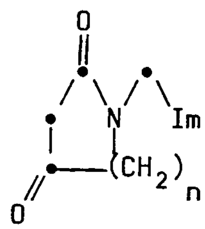

(XII)

or a protected derivative thereof, in a suitable solvent such as an aqueous alcohol (e.g. methanol), and at a temperature of, for example, from 20 to 100 °C.

A protected derivative of a compound of formula (XII) may for example have the keto carbonyl group protected (e.g. as an enol ether). It will be appreciated that when a compound of formula (XII) is used in which the keto carbonyl group is protected, it may be necessary to remove the protecting group in order for reaction to occur with the compound of formula (XI). Deprotection may be carried out by conventional methods, for example by acidic hydrolysis (e.g. using dilute sulphuric or hydrochloric acid). If desired, deprotection may be effected in situ.

Compounds of formula (XII) may be prepared, for example, by reacting a compound of formula (XIII):

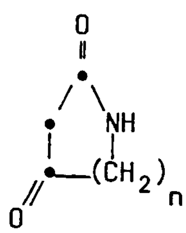

(XIII)

or a protected derivative thereof, with a compound of formula (III) wherein L is as defined previously, or a protected derivative thereof, using the conditions described in process (A).

Compounds of formula (V) wherein W represents a halogen atom and Y represents a bond may be prepared, for example, by reacting a compound of formula (XIV):

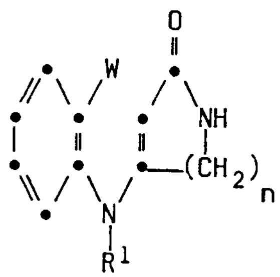

(XIV)

wherein W represents a halogen atom, or a protected derivative thereof, with a compound of formula (III) wherein L is as defined previously, or a protected derivative thereof, using the conditions described in process (A).

Compounds of formula (XIV) may be prepared by reacting a compound of formula (XV):

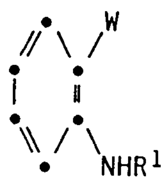

(XV)

with a compound of formula (XIII), at an elevated temperature.

Compounds of formula (VI) may be prepared, for example, by reacting a compound of formula (II), or a protected derivative thereof, with a compound of formula (XVI):

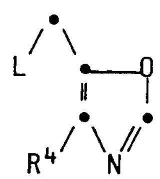

(XVI)

wherein L is as defined previously, using the conditions described in process (A).

Compounds of formula (VII) wherein G represents a halogen atom may be prepared, for example by reacting a compound of formula (VII) wherein G represents a hydrogen atom, or a protected derivative thereof, with an appropriate halogen and alkali metal halide (e.g. iodine and potassium iodide), in a suitable solvent such as an aqueous alcohol (e.g. aqueous ethanol).

Compounds of formula (VII) wherein G represents a hydrogen atom may be prepared, for example, by reacting a compound of formula (XVII):

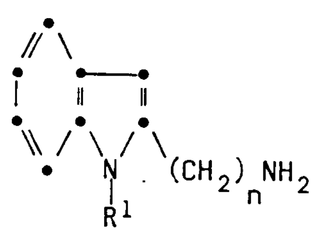

(XVII)

or a protected derivative thereof, with a compound of formula (III) wherein L is as defined previously, or a protected derivate thereof, using the conditions described in process (A).

Compounds of formula (III) and protected derivatives thereof, are either known, or may be prepared, for example, by the methods described in German Offenlegungsschrift No. 3740352.

Compounds of formula (IX) may be prepared, for example, by the method or methods analogous to that described by H. Iida et al. in J. Org. Chem., 1980, 45, 2938.

Compounds of formulae (XI), (XIII), (XV), (XVI) and (XVII) are either known, or may be prepared from known compounds by conventional procedures.

Where it is desired to isolate a compound of the invention as a salt, for example a physiologically acceptable salt, this may be achieved by reacting the compound of formula (I) in the form of the free base with an appropriate acid, preferably with an equivalent amount, in a suitable solvent such as an alcohol (e.g. ethanol or methanol), an aqueous alcohol (e.g. aqueous ethanol), a halogenated hydrocarbon (e.g. dichloromethane), an ester (e.g. ethyl acetate) or an ether (e.g. tetrahydrofuran).

Physiologically acceptable salts may also be prepared from other salts, including other physiologically acceptable salts, of the compound of formula (I) using conventional methods.

Individual enantiomers of the compounds of the invention may be obtained by resolution of a mixture of enantiomers (e.g. a racemic mixture) using conventional means, such as an optically active resolving acid; see for example 'Stereochemistry of Carbon Compounds' by E.L.Eliel (McGraw Hill, 1962) and 'Tables of Resolving Agents' by S. H. Wilen.

The methods described above for preparing the compounds of the invention may be used for the introduction of the desired groups at any stage in the stepwise formation of the required compounds, and it will be appreciated that these methods can be combined in different ways in such multi-stage processes. The sequence of the reactions in multi-stage processes should of course be chosen so that the reaction conditions used to not affect groups in the molecule which are desired in the final product.

The invention is further illustrated by the following Intermediates and Examples. All temperatures are in °C. Thin layer chromatography (t.l.c.) was carried out on silica, and flash column chromatography (FCC) on silica (Merck 9385). Solvent System A as used for chromatography denotes dichloromethane:ethanol:0.88 ammonia solution. Organic extracts were dried, where indicated, over magnesium sulphate or sodium sulphate. The following abbreviations are used: DMF - dimethylformamide; THF - tetrahydrofuran; DME - dimethoxyethane. $^1$H-N.m.r. spectra were obtained at 250MHz for dilute solutions in $d_6$-dimethyl sulphoxide.

## Intermediate 1

### 4-(Chloromethyl)-1-(triphenylmethyl)-1H-imidazole

Thionyl chloride (0.82g) was added over 1 min. to a stirred suspension of 1-(triphenylmethyl)-1H-imidazole-4-methanol (1.3g) in a mixture of dichloromethane (50mℓ) and DMF (1.0mℓ) at 23°. The solution so obtained was stirred for 15 min. and extracted with 8% sodium bicarbonate solution (80mℓ). The organic phase was washed with water (50mℓ), dried and evaporated to give an oil which solidified. The solid was slurried in hexane and filtered to give the title compound (1.28g), m.p. 139-141°.

## Intermediate 2

### 4-Formyl-N,N-dimethyl-5-propyl-1H-imidazole-1-sulphonamide

Dimethylsulphamoyl chloride (0.67mℓ) was added to a stirred solution of 5-propyl-1H-imidazole-4-carboxaldehyde (860mg) and triethylamine (0.87mℓ) in dry dichloromethane (10mℓ) under nitrogen. The solution was heated at reflux for 24h, allowed to cool, poured into water (50mℓ) and extracted with dichloromethane (3x25mℓ). The combined, dried organic extracts were evaporated to give an oil (1.9g) which was purified by FCC eluting with ethyl acetate:hexane (1:1) to give the title compound (500mg), m.p. 57-58°.

Intermediate 3

4-(Hydroxymethyl)-N,N-dimethyl-5-propyl-1H-imidazole-1-sulphonamide

Sodium borohydride (139mg) was added to a stirred solution of 4-formyl-N,N-dimethyl-5-propyl-1H-imidazole-1-sulphonamide (450mg) in absolute ethanol (5mℓ) under nitrogen. After 3h the mixture was poured into water (30mℓ) and extracted with dichloromethane (3x15mℓ). The combined, dried organic extracts were evaporated to give a solid (425mg) which was triturated with ether (2x10mℓ) to give the title compound - (350mg), m.p. 86-88°.

Intermediate 4

4-(Chloromethyl)-N,N-dimethyl-5-propyl-1H-imidazole-1-sulphonamide

A solution of thionyl chloride (0.12mℓ) in dry dichloromethane (1.2mℓ) was added dropwise to a cold (0°) stirred solution of 4-(hydroxymethyl)-N,N-dimethyl-5-propyl-1H-imidazole-1-sulphonamide (340mg) in dry dichloromethane (7.5mℓ) under nitrogen. After 1.5h the solution was washed with 8% sodium bicarbonate solution (2x15mℓ) and the aqueous phase was extracted with dichloromethane (2x10mℓ). The combined organic extracts were washed with water (15mℓ), dried and evaporated to give the title compound (180mg) as an oil, t.l.c. (ethyl acetate) Rf 0.68.

Intermediate 5

3,4-Dihydro-4-methylcyclopent[b]indol-1(2H)-one oxime

3,4-Dihydro-4-methylcyclopent[b]indol-1-(2H)-one (1.7g) and hydroxylamine hydrochloride (1.925g) in pyridine were heated at 60° for 18h and cooled. The reaction mixture was evaporated in vacuo to a residue to which was added 8% sodium bicarbonate (150mℓ). Extraction with ethyl acetate (300mℓ) produced a suspension in the organic layer; this layer and associated solid was separated from the aqueous layer. The aqueous layer was re-extracted with ethyl acetate (250mℓ). The combined organic extracts (and suspended solid) were evaporated to a residue, boiled with a mixture of ethanol (150mℓ) and methanol (150mℓ) and cooled to ca. 50°. The residue was adsorbed from this solution on to FCC silica and applied to an FCC column. Elution with ethyl acetate/3-10% methanol provided the title compound (1.69g), m.p. 219-224° (decomp.).

Intermediate 6

2,3,4,5-Tetrahydro-5-methyl-1H-pyrido[4,3-b]indol-1-one

3,4-Dihydro-4-methylcyclopent[b]indol-1(2H)-one oxime (1.53g), polyphosphoric acid (40g) and dioxan (15mℓ) were heated at 110-120° for 2.2h under nitrogen. The reaction mixture was cooled, and treated with 2N sodium carbonate solution (1ℓ). The suspension was extracted with ethyl acetate (4x400mℓ) and the combined extracts were dried. Evaporation gave a solid (1.43g) which was recrystallised from ethyl acetate/cyclohexane. This solid was purified by FCC, eluting with System A (200:10:1) to give a solid (1.26 g) which was recrystallised from ethanol to provide the title compound (960 mg), m.p. 234-238°.

Intermediate 7

## 3,4,5,6-Tetrahydro-6-methylazepino[4,3-b]indol-1(2H)-one

1,2,3,9-Tetrahydro-9-methyl-4H-carbazol-4-one oxime (24g) and polyphosphoric acid (600g) in dioxan (500mℓ) were treated according to the method described for Intermediate 6. The solid (22g) obtained by evaporation of the organic extracts was recrystallised from ethyl acetate (300mℓ) to give a solid (19.2 g). This was purified by FCC eluting with System A (200:8:1) to give the title compound (5.5g), m.p. 212-215°.

Intermediate 8

## 5,6-Dihydro-4-(phenylamino)-1(2H)-pyridinone

A mixture of 2,4-dioxopiperidine (1.13g) and aniline (930mg) was heated at 120° under a stream of nitrogen for 15 min. The resultant solid was triturated with ether and filtered off to give the title compound (1.74g), m.p. 235-238°.

Intermediate 9

## 2,3,4,5-Tetrahydro-1H-pyrido[4,3-b]indol-1-one

A solution of 5,6-dihydro-4-(phenylamino)-1(2H)-pyridinone (1.5g) and palladium acetate (150mg) in dry DMF (50mℓ) was treated with cupric acetate (3.2g) and the resulting mixture was heated under nitrogen at 120-130° for 1.5 h. The mixture was then concentrated in vacuo to give a solid which was triturated with 2N hydrochloric acid (250mℓ). The acid was decanted, and the remaining solid was extracted with ethyl acetate for 18h. The decanted acid was basified with 2N sodium hydroxide and extracted with ethyl acetate (3x100mℓ). These organic extracts were combined with the previous ethyl acetate extracts and adsorbed onto silica. Purification by FCC eluting with System A (100:8:1) gave the title compound (874mg), m.p. 212-215°.

Intermediate 10

## 2,3,4,5-Tetrahydro-5-[(phenylmethoxy)methyl]-1H-pyrido[4,3-b]indol-1-one

A solution of 2,3,4,5-tetrahydro-1H-pyrido[4,3-b]]indol-1-one (1.12g) in dry DMF (60mℓ) was treated with sodium hydride (60% dispersion in oil; 480mg) and the resulting mixture was stirred under nitrogen until effervescence ceased. The mixture was then cooled to 0° and benzyl (chloromethyl) ether (10% w/v solution in DMF; 0.835ml) was added over 10 min. Stirring was continued for a further 5 min and then water (10mℓ) was added. The reaction mixture was concentrated in vacuo to give an oil which was dissolved in ethyl acetate (100mℓ) and washed with water (3x100mℓ). The organic phase was dried and adsorbed onto FCC silica. Purification by FCC eluting with System A (150:8:1) gave the title compound (1.1g), m.p. 133-135°.

Intermediates 11 to 14 were prepared in a similar manner to Intermediate 10, i.e. by treating 2,3,4,5-tetrahydro-1H-pyrido[4,3-b]indol-1-one with sodium hydride followed by an appropriate alkylating agent. Isolation and purification of the products were as described for Intermediate 10 unless otherwise stated.

Intermediate 11

5-Ethyl-2,3,4,5-tetrahydro-1H-pyrido[4,3-b]indol-1-one

2,3,4,5-Tetrahydro-1H-pyrido[4,3-b]indol-1-one (931mg) was treated with sodium hydride (60% dispersion in oil; 400mg) and was then stirred with ethyl iodide (10% v/v solution in DMF; 4ml) to give the title compound (758mg), m.p. 203-204.5°.

Intermediate 12

2,3,4,5-Tetrahydro-5-(1-methylethyl)-1H-pyrido[4,3-b]indol-1-one

2,3,4,5-Tetrahydro-1H-pyrido[4,3-b]indol-1-one (931mg) was treated with sodium hydride (73% dispersion in oil; 328 mg) and was then stirred with 2-bromopropane (615mg) at room temperature for 72 h. Purification by FCC eluting with System A (200:8:1) gave a foam (324mg) which was further purified by recrystallisation from ethyl acetate: hexane (1:1) to give the title compound (294mg), t.l.c. (System A, 100:8:1) Rf 0.58.

Intermediate 13

2,3,4,5-Tetrahydro-5-(phenylmethyl)-1H-pyrido[4,3-b]indol-1-one

2,3,4,5-Tetrahydro-1H-pyrido[4,3-b]indol-1-one (559mg) was treated with sodium hydride (73% dispersion in oil; 197mg) and was then stirred with benzyl bromide (513 mg) at room temperature for 30 min. Purification by FCC eluting with dichloromethane: ethanol (80:1) gave the title compound (347 mg), m.p. 209-212°.

Intermediate 14

5-(Cyclopentylmethyl)-2,3,4,5-tetrahydro-1H-pyrido[4,3-b]indol-1-one

2,3,4,5-Tetrahydro-1H-pyrido[4,3-b]indol-1-one (950mg) was treated with sodium hydride (60% dispersion in oil; 408mg) and was then stirred with cyclopentanemethanol, methanesulphonate (909mg) at room temperature for 7 days. The solid (570mg) obtained by FCC was further purified by slow evaporation from a solution in methanol to give the title compound, m.p. 179-181°.

Intermediate 15

2,3,4,5-Tetrahydro-2-[[5-methyl-1-(triphenylmethyl)-1H-imidazol-4-yl]methyl]-1H-pyrido[4,3-b]indol-1-one

A solution of triphenylmethyl chloride (3.36g) in dry DMF (40mℓ) was added dropwise to a stirred solution of 2,3,4,5-tetrahydro-2-[(5-methyl-1H-imidazol-4-yl)methyl]-1H-pyrido[4,3-b]indol-1-one (2.8g) in dry DMF (50mℓ) containing triethylamine (1.52g). When addition was complete the mixture was stirred overnight. The mixture was then poured into water (1000ml) and the resulting suspension was extracted with ethyl acetate (3 x 300mℓ). The combined organic extracts were washed with water (2 x 500mℓ), dried and concentrated onto silica. FCC eluting with System A (100:8:1) gave the title compound (4.3g), m.p. 235-236°.

Intermediate 16

2,3,4,5-Tetrahydro-5-methyl-2-[[1-(triphenylmethyl)-1H-imidazol-4-yl]methyl]-1H-pyrido[4,3-b]indol-1-one

A mixture of 2,3,4,5-tetrahydro-5-methyl-1H-pyrido[4,3-b]indol-1- one (0.3g) and sodium hydride (80% dispersion in oil; 0.05g) in dry DMF (5mℓ) was stirred under nitrogen at 50° until hydrogen evolution ceased (ca. 0.5h). The mixture was cooled to 40° and a solution of 4-(chloromethyl)-1-(triphenylmethyl)-1H-imidazole (0.53 g) in dry THF (3mℓ) was added. The mixture was stirred at 40° to 23° over 2h, poured into water (100mℓ) and extracted with dichloromethane (3x100mℓ). The dried organic phase was evaporated to give a semi-solid which was purified by FCC eluting with dichloromethane:ethyl acetate:triethylamine (50:50:1) to give a solid. This was slurried in hexane and filtered to give the title compound (0.37g), m.p. 205-210° (decomp.).

Intermediate 17

2,5-Dihydro-5-methyl-1H-pyrido[4,3-b]indol-1-one

A mixture of 2,3,4,5-tetrahydro-5-methyl-1H-pyrido[4,3-b]indol-1-one (500mg) and 10% palladium oxide on carbon catalyst (50% aqueous paste; 250mg) was heated at 320° for 10 min. The cooled solid was triturated with ethanol (ca. 100mℓ), filtered and the resulting filtrate was evaporated to give the title compound (470mg), m.p. 242.5°.

Intermediate 18

2,5-Dihydro-5-methyl-2-[(5-methyl-1H-imidazol-4-yl)methyl]-1H-pyrido[4,3-b]indol-1-one maleate

Sodium hydride (73% dispersion in oil; 80mg) was added to a stirred suspension of 2,5-dihydro-5-methyl-1H-pyrido[4,3-b]indol-1-one (440mg) in dry dimethoxyethane (25mℓ) under nitrogen and the mixture was heated at 50° for 6h. 4-(Chloromethyl)-5-methyl-1-(triphenylmethyl)-1H-imidazole (910mg) was then added, and stirring was continued at 50° for 20h. Water (4.5mℓ) and acetic acid (4.5mℓ) were added and the solution was heated at reflux for 5h. The mixture was poured into 8% sodium bicarbonate solution (80mℓ) and extracted with dichloromethane:ethanol (10:1; 3x40mℓ). The combined, dried organic extracts were evaporated to give a solid (ca. 1.5g) which was purified by FCC eluting with System A (200:10:1) to give the free base of the title compound as a solid (384mg). A sample of this solid (100mg) was dissolved in absolute ethanol (20mℓ) and treated with a solution of maleic acid (40mg) in absolute ethanol (1mℓ). The solvent was removed in vacuo and the residue was triturated with dry ether (3x20mℓ) to give a solid (115mg) which was re-crystallised from methanol-ethyl acetate to give the title compound (40mg), m.p. 166-168°.

Intermediate 19

5,6-Dihydro-4-methoxy-1-[[5-methyl-1-(triphenylmethyl)-1H-imidazol-4-yl]methyl]-2(1H)-pyridinone

Sodium hydride (80% dispersion in oil; 360mg) was suspended in dry DME (50mℓ) under nitrogen and 5,6-dihydro-4-methoxy-2(1H)-pyridinone (1.27g) in dry DME (20mℓ) was added slowly. The resulting suspension was stirred at 20° for 1h. 4-(Chloromethyl)-5-methyl-1-(triphenylmethyl)-1H-imidazole (3.72g) in dry DME (50mℓ) was added, and after the initial reaction had subsided the mixture was heated to 50° for 4h and then cooled. Methanol (5mℓ) was added dropwise, and the solvent was removed in vacuo. 8% Aqueous sodium bicarbonate solution (300mℓ) was added to the residue and the resulting solution was extracted with dichloromethane (2x300mℓ), dried and evaporated in vacuo to leave an oil which was purified by FCC eluting with System A (200:8:1) to give the title compound (2.84g), m.p. 181-184°.

Intermediate 20

2,4-Dioxo-1-[(5-methyl-1H-imidazol-4-yl)methyl]piperidine

To a solution of 5,6-dihydro-4-methoxy-1-[[5-methyl-1-(triphenylmethyl)-1H-imidazol-4-yl]methyl]-2(1H)-pyridinone (500mg) in THF (4mℓ) was added hydrochloric acid (5M; 1mℓ), and the mixture was stirred at 50° for 1h. The solvent was removed in vacuo, triethylamine (1mℓ) was added, and the mixture was again evaporated to dryness. FCC of the residue eluting with ethyl acetate:methanol:triethylamine (8:4:1) gave the title compound (139mg), m.p. 100-106° (decomp.).

Intermediate 21

5,6-Dihydro-1-[(5-methyl-1H-imidazol-4-yl)methyl]-4-(2-methyl-2-phenylhydrazino)-2(1H)-pyridinone

2,4-Dioxo-1-[(5-methyl-1H-imidazol-4-yl)methyl]piperidine (20mg) was dissolved in ethanol (2mℓ) and N-methylphenylhydrazine (26mg) was added. The mixture was stirred for 1h and the solvent was removed in vacuo. The residue was purified by FCC eluting with System A (75:8:1) to give the title compound (24mg) as a solid, t.l.c. (System A, 75:8:1) Rf 0.27.

Intermediate 22

N,N,5-Trimethyl-4-[[(trimethylsilyl)oxy]methyl]-1H-imidazole-1-sulphonamide

A suspension of 4-(hydroxymethyl)-5-methylimidazole hydrochloride (14.9g) in dry dichloromethane (500mℓ) containing triethylamine (50g) was treated with trimethylsilyl chloride (21.7g) and the reaction mixture was stirred at room temperature overnight. Dimethylsulphamoyl chloride (14.3g) was added and the reaction mixture was again stirred at room temperature overnight. The resulting suspension was filtered and the collected solid was washed with dichloromethane (100mℓ). The filtrate was concentrated onto silica and purification by FCC eluting with hexane:ether (4:1) gave the title compound as an oil (7.2g), t.l.c. (ether) Rf 0.5.

Intermediate 23

4-(Hydroxymethyl)-N,N,5-trimethyl-1H-imidazole-1-sulphonamide

A solution of N,N,5-trimethyl-4-[[(trimethylsilyl)oxy]methyl]-1H-imidazole-1-sulphonamide (2.59g) in dry THF (50mℓ) was treated with a solution of tetrabutylammonium fluoride (1M solution in THF; 10ml). and the THF was immediately removed in vacuo. The residue was partitioned between water (100mℓ) and dichloromethane (100mℓ) and the aqueous layer was extracted with dichloromethane (100mℓ). The combined, dried organic fractions were concentrated to give the title compound (1.63g) as a solid, m.p. 134-136°.

Intermediate 24

4-(Chloromethyl)-N,N,5-trimethyl-1H-imidazole-1-sulphonamide

A suspension of 4-(hydroxymethyl)-N,N,5-trimethyl-1H-imidazole-1-sulphonamide (2.86g) in dry dichloromethane (200mℓ) containing DMF (0.5mℓ) was treated dropwise with a solution of thionyl chloride (1.178g) in dichloromethane (10mℓ). The reaction mixture was cooled in ice during the addition and blanketed with nitrogen. When addition was complete (ca. 5min), stirring was continued at 0° for a further 30 min. Water (200mℓ) was then added and the organic phase was separated, washed with 8% sodium bicarbonate (100mℓ), dried and concentrated to give the title compound (2.3g) as a solid, m.p. 115-118°.

Intermediate 25

5,6-Dihydro-4-[(2-iodophenyl)methylamino]-2(1H)-pyridinone

A mixture of 2-iodo-(N-methyl)aniline (1.17g) and 2,4-dioxopiperidine (565mg) was heated under a stream of nitrogen for 7h at 110-120°. After cooling the reaction mixture was dissolved in methanol and the solution was adsorbed onto FCC silica. Purification by FCC eluting with System A (150:8:1) gave the title compound (1.03g), m.p. 163-164°.

Intermediate 26

N,N,5-Trimethyl-4-[[1,2,3,6-tetrahydro-4-[(2-iodophenyl)-methylamino]-6-oxo-1-pyridinyl]methyl]-1H-imidazole-1-sulphonamide

A suspension of 5,6-dihydro-4-[(2-iodophenyl)methylamino]-2(1H)-pyridinone (984mg) in dry DME (50mℓ) was treated with sodium hydride (60% dispersion in oil; 140mg), and the mixture was stirred under nitrogen for 6h. 4-(Chloromethyl)-N,N,5-trimethyl-1H-imidazole-1-sulphonamide (832mg) was then added and the resulting mixture was stirred at 60° overnight. After cooling the reaction mixture was poured into water (100mℓ), and the mixture was extracted with ethyl acetate (2x50mℓ). The combined, dried organic extracts were concentrated, and the resultant solid was purified by FCC eluting with System A (150:8:1) to give the title compound (712mg), t.l.c. (System A, 150:8:1) Rf 0.41.

Intermediate 27

N,N,5-Trimethyl-4-[(2,3,4,5-tetrahydro-5-methyl-1-oxo-1H-pyrido[4,3-b]indol-2-yl)methyl]-1H-imidazole-1-sulphonamide

A solution of dimethylsulphamoyl chloride (0.107mℓ) in dry dichloromethane was added to a stirred solution of 2,3,4,5-tetrahydro-5-methyl-2-[(5-methyl-1H-imidazol-4-yl)methyl]-1H-pyrido[4,3-b]indol-1-one (0.294g) and triethylamine (0.2mℓ) in dry dichloromethane (30mℓ) under nitrogen, and the mixture was heated at reflux for ca. 24h. After cooling the solution was concentrated onto FCC silica and purified by FCC eluting with System A (150:8:1) to give an oil. This oil was triturated with ether to give a solid which was further purified by slow evaporation from a solution in ethyl acetate to give the title compound (122 mg), m.p. 194-196°, t.l.c. (System A, 100:8:1) Rf 0.43.

Intermediate 28

Phenylmethyl 5-methyl-4-[(2,3,4,5-tetrahydro-5-methyl-1-oxo-1H-pyrido[4,3-b]indol-2-yl)methyl]-1H-imidazole-1-carboxylate

A solution of benzyl chloroformate (0.28mℓ) in dichloromethane (10mℓ) was added to a stirred solution of 2,3,4,5-tetrahydro-5-methyl-2-[(5-methyl-1H-imidazol-4-yl)methyl]-1H-pyrido[4,3-b]indol-1-one (294mg) and triethylamine (0.4mℓ) in dichloromethane (30mℓ) at 20° under nitrogen, and the mixture was stirred overnight. It was then concentrated onto FCC silica and purified by FCC eluting with System A (200:8:1) to give the title compound (62mg), t.l.c. (System A, 100:8:1) Rf 0.50.

Intermediate 29

2,3,4,5-Tetrahydro-2-[[1-(methoxymethyl)-5-methyl-1H-imidazol-4-yl]-methyl]-5-methyl-1H-pyrido[4,3-b]indol-1-one and 2,3,4,5-Tetrahydro-2-[[1-(methoxymethyl)-4-methyl-1H-imidazol-5-yl]methyl]-5-methyl-1H-pyrido[4,3-b]indol-1-one

A solution of chloromethyl methyl ether (0.26mℓ) in dichloromethane (10mℓ) was added to a stirred solution of 2,3,4,5-tetrahydro-5-methyl-2-[(5-methyl-1H-imidazol-4-yl)methyl]-1H-pyrido[4,3-b]indol-1-one (500mg) and triethylamine (0.49mℓ) in dichloromethane (50mℓ) at 20° under nitrogen, and the solution was stirred for 4 days. It was then partitioned between dichloromethane (50mℓ) and sodium bicarbonate solution (2x50mℓ). The organic extract was dried, concentrated onto FCC silica, and then purified by FCC eluting with System A (100:8:1) to give the title compounds (139mg). A portion of the title compounds (64mg) was taken up in hot ethyl acetate and purified by slow evaporation from ethyl acetate to give the title compounds.

| | | | |
|---|---|---|---|
| Analysis Found: | C,67.3; | H,6.9; | N,16.5; |
| $C_{19}H_{22}N_4O_2$ requires | C,67.4; | H,6.6; | N,16.6%. |

Intermediate 30

2,3,4,5-Tetrahydro-5-methyl-2-[(4-methyloxazol-5-yl)methyl]-1H-pyrido[4,3-b]-indol-1-one

Sodium hydride (60% dispersion in oil; 600mg) was added to a stirred suspension of 2,3,4,5-tetrahydro-5-methyl-1H-pyrido[4,3-b]indol-1-one (1.5g) in dry DME (150mℓ) and then the mixture was stirred at 60° for 5h under nitrogen. 5-Chloromethyl-4-methyloxazole (1.2g) was added and the mixture was stirred overnight. A further quantity of sodium hydride (60% dispersion in oil; 600mg) was added and the mixture was stirred at 60° for 4h, then cautiously treated with water (100mℓ). The mixture was extracted with dichloromethane containing methanol (ca. 1%) (3x100mℓ) and the combined extracts were evaporated. The residue was purified by FCC eluting with System A (100:8:1) to give the title compound (300mg) as a solid, t.l.c. (System A, 100:8:1) Rf 0.4.

Intermediate 31

N-[(1-Methyl-1H-indol-2-yl)ethyl]trifluoroacetamide

A solution of 2-(1-methyl-1H-indol-2-yl)ethanamine (3.48g) in dry dlchloromethane (50mℓ) containing triethylamine (2.53g) was cooled in an ice bath, and trifluoroacetic anhydride (5.25g) was added dropwise over 15 min. The mixture was then allowed to warm to room temperature and stirred for an additional 3h. After this time the reaction mixture was poured into water (100mℓ), the organic phase was separated, and the aqueous phase was washed with dichloromethane (2x50mℓ). The combined, dried organic extracts were concentrated onto FCC silica and purification by FCC eluting with ether gave the title compound (4.2g) as a solid. A sample of this compound was further purified by slow evaporation from a solution in dichloromethane, m.p. 124-126°.

Intermediate 32

N,N,5-Trimethyl-4-[[(1-methyl-1H-indol-2-yl)-N-trifluoroacetylamino]-ethyl]imidazole-1-sulphonamide

A solution of N-[(1-methyl-1H-indol-2-yl)ethyl]trifluoroacetamide (2.7g) in dry DMF (100mℓ) was treated with sodium hydride (60% dispersion in oil; 480mg), and the mixture was stirred at room temperature for 30 min. 4-(Chloromethyl)-N,N,5-trimethyl-1H-imidazole-1-sulphonamide (2.37g) was then added and the mixture was stirred at room temperature overnight. After this time the reaction mixture was poured into water (500mℓ) and the resulting suspension was extracted with ethyl acetate (2x100mℓ). The combined organic extracts were washed with water (5x250mℓ), dried and adsorbed onto FCC silica. Purification by FCC eluting with System A (150:8:1) gave the title compound (1.9g), m.p. 156-158°.

Intermediate 33

4-[[[(1-Methyl-1H-indol-2-yl)ethyl]amino]methyl]-N,N,5-trimethyl-1H-imidazole-1-sulphonamide

A mixture of N,N,5-trimethyl-4-[[(1-methyl-1H-indol-2-yl)-N-trifluoro-acetylamino]ethyl]imidazole-1-sulphonamide (260mg), methanol (10mℓ) and saturated aqueous potassium carbonate solution (5mℓ) was heated to 60° for 1.5h. After cooling the mixture was poured into water (50mℓ) and the mixture was extracted with ethyl acetate (2x50mℓ). The combined, dried organic extracts were concentrated onto FCC silica and purified by FCC eluting with System A (150:8:1) to give the title compound (143mg) as an oil, t.l.c. (System A, 100:8:1) Rf 0.51.

Intermediate 34

## 4-[[[(3-Iodo-1-methyl-1H-indol-2-yl)ethyl]trifluoroacetylamino]-methyl]-N,N,5-trimethyl-1H-imidazole-1-sulphonamide

A solution of 4-[[[(1-methyl-1H-indol-2-yl)ethyl]amino]methyl]-N,N,5-trimethyl-1H-imidazole-1-sulphonamide (471mg) in methanol (25mℓ) containing potassium carbonate (138mg) was treated with a solution of iodine (254mg) and potassium iodide (166mg) in water (30mℓ) over 30 min. When addition was complete the reaction mixture was stirred for a further 2h. After this time additional methanol was removed in vacuo and the resulting suspension was extracted with ethyl acetate (3x25mℓ). The combined organic extracts were concentrated onto FCC silica and purified by FCC eluting with System A (150:8:1) to give the title compound (367mg), m.p. 141-143°.

Intermediate 35

## 4-[[[(3-Iodo-1-methyl-1H-indol-2-yl)ethyl]amino]methyl]-N,N,5-trimethyl-1H-imidazole-1-sulphonamide

4-[[[(3-Iodo-1-methyl-1H-indol-2-yl)ethyl]trifluoroacetylamino]methyl]-N,N,5-trimethyl-1H-imidazole-1-sulphonamide (199mg) was deprotected according to the method described in Intermediate 33 to give the title compound (50mg) as an oil, t.l.c. (System A, 150:8:1) Rf 0.51.

Example 1

## 2,3,4,5-Tetrahydro-5-methyl-2-[(5-methyl-1H-imidazol-4-yl)methyl]-1H-pyrido[4,3-b]indol-1-one maleate.

A mixture of 2,3,4,5-tetrahydro-5-methyl-1H-pyrido[4,3-b]indol-1-one (0.6g) and ca. 78% sodium hydride dispersion in mineral oil (0.109g) in dry DMF (15mℓ) was stirred under nitrogen at 50° until hydrogen evolution ceased (ca. 1.5h). The mixture was cooled to 40° and a solution of 4-(chloromethyl)-5-methyl-1-(triphenylmethyl)-1H-imidazole (1.12g) in dry THF (15mℓ) was added. The reaction was then stirred at 40° for 3h, at 20° for 16h and a further portion of 4-(chloromethyl)-5-methyl-1-(triphenylmethyl)-1H-imidazole (1.12g) in dry THF (15mℓ) was added. The resulting mixture was heated at 40° for 3h, quenched with water (20mℓ) and acetic acid (20mℓ), and heated at 100° for 2h. The mixture was then concentrated in vacuo to ca. 60mℓ, diluted with 1M hydrochloric acid (40mℓ) and washed with ethyl acetate (3 x 50mℓ). The organic phase was discarded and the acidic aqueous phase was basified (pH9) with potassium carbonate and extracted with ethyl acetate: ethanol (20:1, 3 x 100mℓ). The extracts were combined, dried and evaporated to give a brown gum (ca. 1g). This gum was adsorbed onto silica and purified by FCC eluting with System A (100:8:1) to give a pale brown solid (0.8g) m.p. 238-240° (decomp). This solid was dissolved in a mixture of hot ethanol and methanol (1:1; 100mℓ) and treated with an ethanolic solution of maleic acid (318 g). The resulting solution was concentrated to ca. 20mℓ and diluted with dry diethyl ether (ca. 8mℓ) to precipitate the title compound (0.75g) as an off-white solid, m.p. 160-162°.

| | | | |
|---|---|---|---|
| Analysis Found: | C,61.6; | H,5.5; | N,13.6; |
| $C_{17}H_{18}N_4O.C_4H_4O_4$ requires | C,61.5; | H,5.4; | N,13.8%. |

Example 2

3,4,5,6-Tetrahydro-6-methyl-2-[(5-methyl-1H-imidazol-4-yl)methyl]-azepino[4,3-b]indol-1(2H)-one maleate

3,4,5,6-Tetrahydro-6-methylazepino[4,3-b]indol-1(2H)-one (0.64g) was treated with sodium hydride (ca. 75-80% dispersion in oil; 0.108g) and was then stirred with 4-(chloromethyl)-5-methyl-1-(triphenylmethyl)-1H-imidazole as described in Example 1. The reaction mixture was then poured into water (300mℓ) and extracted with dichloromethane (4x250mℓ). The combined, dried organic extracts were evaporated to give a semi-solid (ca. 1.8g) which was purified by FCC eluting with System A (200:8:1) to give a gum (0.7g). This gum (0.7g) was dissolved in a mixture of acetic acid, THF and water (1:1:1; ca. 70mℓ) and heated on a steam bath for 1h. Work-up as described in Example 1 gave a gum (0.22g) which was purified by FCC eluting with System A (200:8:1) to give a solid (0.11g). Maleate formation gave a gum which was dried in vacuo to give a foam which was triturated with a mixture of ether and ethanol (50:1; ca. 25mℓ) to give the title compound (0.145g) as a solid, m.p. 132-133°.
$^1$H-N.m.r. indicated 0.39mol of ethanol present.
Water Analysis Found 0.583% w/w≡0.14mol $H_2O$.

| | | | |
|---|---|---|---|
| Analysis Found: | C,61.4; | H,5.7; | N,12.6; |
| $C_{18}H_{20}N_4O$. $C_4H_4O_4$.0.39EtOH. 0.14$H_2O$ requires | C,61.4; | H,6.0; | N,12.6%. |

Example 3

2,3,4,5-Tetrahydro-2-[(5-methyl-1H-imidazol-4-yl)methyl]-5-[(phenylmethoxy)methyl]-1H-pyrido[4,3-b]indol-1-one maleate

A suspension of 2,3,4,5-tetrahydro-5-[(phenylmethoxy)methyl]-1H-pyrido[4,3-b]indol-1-one (920mg) in dry DME (75mℓ) was treated with sodium hydride (60% dispersion in oil; 180mg) under nitrogen and the reaction mixture was stirred at 60° for 6 h. 4-(Chloromethyl)-5-methyl-1-(triphenylmethyl)-1H-imidazole (1.11g) was then added and the mixture was stirred at 60° overnight. Acetic acid (10mℓ), water (10mℓ) and THF (10mℓ) were then added and the resulting solution was heated at reflux for 6 h. After cooling, 2N sodium hydroxide (100mℓ) was added and the resulting suspension was extracted with dichloromethane (3 x 100mℓ). The combined, dried organic extracts were adsorbed onto FCC silica, and FCC eluting with System A (150:8:1) gave the free base of the title compound (1.08g) as a foam. A small amount of this compound (200mg) was dissolved in methanol (30mℓ) and the resulting solution was treated with maleic acid (58mg). The solution was heated for 10 min., cooled, and dry ether was added to precipitate the title compound (170 mg), m.p. 165-168°.
Water Analysis Found 0.22% w/w ≡ 0.06 mol $H_2O$.

| | | | |
|---|---|---|---|
| Analysis Found: | C,64.5; | H,5.6; | N,10.7; |
| $C_{24}H_{24}N_4O.C_4H_4O_4$. 0.06 $H_2O$ requires | C,65.0; | H,5.5; | N,10.8%. |

Examples 4 to 7 were prepared in a similar manner to Example 3.

Example 4

5-Ethyl-2,3,4,5-tetrahydro-2-[(5-methyl-1H-imidazol-4-yl)methyl]-1H-pyrido[4,3-b]indol-1-one maleate

5-Ethyl-2,3,4,5-tetrahydro-1H-pyrido[4,3-b]indol-1-one (500mg) was treated with sodium hydride (60% dispersion in oil; 138mg) and was then stirred with 4-(chloromethyl)-5-methyl-1-(triphenylmethyl)-1H-imidazole (927.5mg) to give the free base of the title compound (320mg) as a solid. Maleate formation gave the title compound (380mg), m.p. 175.5-177°.

| | | | |
|---|---|---|---|
| Analysis Found: | C,62.1; | H,5.7; | N,13.0; |
| $C_{18}H_{20}N_4O.C_4H_4O_4$ requires | C,62.2; | H,5.7; | N,13.2%. |

Example 5

2,3,4,5-Tetrahydro-5-(1-methylethyl)-2-[(5-methyl-1H-imidazol-4-yl)methyl]-1H-pyrido[4,3-b]indol-1-one maleate

2,3,4,5-Tetrahydro-5-(1-methylethyl)-1H-pyrido[4,3-b]indol-1-one (228mg) was treated with sodium hydride (60% dispersion in oil; 60mg) and was then stirred with 4-(chloromethyl)-5-methyl-1-(triphenylmethyl)-1H-imidazole (371mg) to give the free base of the title compound (180mg) as a solid. Maleate formation gave the title compound (172mg), m.p. 203-205°.

| | | | |
|---|---|---|---|
| Analysis Found: | C,62.6; | H,6.0; | N,12.6; |
| $C_{19}H_{22}N_4O.C_4H_4O_4$ requires | C,63.0; | H,6.0; | N,12.8%. |

Example 6

2,3,4,5-Tetrahydro-5-(phenylmethyl)-2-[(5-methyl-1H-imidazol-4-yl)-methyl]-1H-pyrido[4,3-b]indol-1-one maleate monohydrate

2,3,4,5-Tetrahydro-5-(phenylmethyl)-1H-pyrido[4,3-b]indol-1-one (960mg) was treated with sodium hydride (73% dispersion in oil; 132mg) and was then stirred with 4-(chloromethyl)-5-methyl-1-(triphenylmethyl)-1H-imidazole (1.3g). The free base of the title compound (571mg) was obtained as a solid by FCC eluting with System A (175:8:1). Maleate formation gave the title compound (420mg), m.p. 198-200°, t.l.c. (System A, 100:8:1) Rf 0.3.

Example 7

**5-(Cyclopentylmethyl)-2,3,4,5-tetrahydro-2-[(5-methyl-1H-imidazol-4-yl)methyl]-1H-pyrido[4,3-b]indol-1-one maleate**

5-(Cyclopentylmethyl)-2,3,4,5-tetrahydro-1H-pyrido[4,3-b]indol-1-one (200mg) was treated with sodium hydride (60% dispersion in oil; 60mg) and was then stirred with 4-(chloromethyl)-5-methyl-1-(triphenylmethyl)-1H-imidazole (280mg). The free base of the title compound was obtained as a solid (96mg) by FCC eluting with System A (200:8:1). Maleate formation gave the title compound (60mg), m.p. 81-83°, t.l.c. (System A, 100:8:1) Rf 0.20.

Example 8

**2,3,4,5-Tetrahydro-5-methyl-2-[(5-propyl-1H-imidazol-4-yl)methyl]-1H-pyrido[4,3-b]indol-1-one maleate**

Sodium hydride (60% dispersion in oil; 25mg) was added to a stirred suspension of 2,3,4,5-tetrahydro-5-methyl-1H-pyrido[4,3-b]indol-1-one (124mg) in dry DME (5mℓ) under nitrogen. The mixture was heated at 50° for 7h and then treated with a solution of 4-(chloromethyl)-N,N-dimethyl-5-propyl-1H-imidazole-1-sulphonamide (165mg) in dry DME (3mℓ) and stirring was continued at 50° for 20h. 2N Hydrochloric acid (5mℓ) was added and the solution was heated at reflux for 6h. The solution was poured into 8% sodium bicarbonate solution (50mℓ) and extracted with dichloromethane (3x25mℓ). The combined, dried organic extracts were evaporated to give a solid (200mg) which was purified by FCC eluting with System A (200:10:1) to give the free base of the title compound (58mg) as a solid. This was dissolved in warm absolute ethanol (5mℓ) and treated with a solution of maleic acid (21mg) in ethanol (0.5mℓ). The solvent was removed in vacuo and the residue was crystallised from ethanol:ether to give the title compound - (58mg), m.p. 137-138°.

| | | | |
|---|---|---|---|
| Analysis Found: | C,62.7; | H,5.9; | N,12.4; |
| $C_{19}H_{22}N_4O.C_4H_4O_4$ requires | C,63.0; | H,6.0; | N,12.8%. |

Example 9

**2,3,4,5-Tetrahydro-N,N-dimethyl-2-[(5-methyl-1H-imidazol-4-yl)methyl]-1-oxo-5H-pyrido[4,3-b]indole-5-carboxamide maleate**

A solution of 2,3,4,5-tetrahydro-2-[[5-methyl-1-(triphenylmethyl)-1H-imidazol-4-yl]methyl]-1H-pyrido[4,3-b]-indol-1-one (261mg) in dry DMF 25mℓ) was treated with sodium hydride (60% dispersion in oil; 30mg) and the mixture was stirred at room temperature under nitrogen for 15 min. N,N-Dimethylcarbamoyl chloride (1M solution in DMF; 1ml) was then added and the solution was stirred at room temperature for an additional 15 min. Water (1mℓ) was cautiously added, and the reaction mixture was then poured into water (100mℓ). The resulting mixture was extracted with ethyl acetate (2 x 50mℓ) and the combined organic extracts were washed with water (2 x 100mℓ) and concentrated to give an oil. The oil was dissolved in a mixture of water (10mℓ), glacial acetic acid (10mℓ) and THF (10mℓ) and the solution was heated at reflux for 1.5 h. After cooling the solution was basified by addition of 2N sodium hydroxide (100mℓ), and the resulting mixture was extracted with ethyl acetate (2 x 75mℓ). The combined, dried organic extracts were adsorbed onto FCC silica and the free base of the title compound (110mg) was obtained by FCC eluting

with System A (100:8:1) as a solid. This was dissolved in dry methanol (10mℓ) and heated with maleic acid (36mg) on a steam bath for 5 min. On cooling, dry ether (3mℓ) was added to precipitate the title compound (105mg), m.p. 161-163°.
Water Analysis Found 1.85% w/w ≡ 0.49 mol $H_2O$.

| | | | |
|---|---|---|---|
| Analysis Found: | C,57.8; | H,5.4; | N,14.3; |
| $C_{19}H_{21}N_5O_2.C_4H_4O_4$. 0.49 $H_2O$ requires | C,68.0; | H,5.5; | N,14.7%. |

Examples 10, 11 and 12 were prepared in a similar manner to Example 9 unless otherwise stated.

Example 10

### 2,3,4,5-Tetrahydro-2-[(5-methyl-1H-imidazol-4-yl)methyl]-5-(methylsulphonyl)-1H-pyrido[4,3-b]indol-1-one maleate

2,3,4,5-Tetrahydro-2-[[5-methyl-1-(triphenylmethyl)-1H-imidazol-4-yl]methyl]-1H-pyrido[4,3-b]indol-1-one (261mg) was treated with sodium hydride (60% dispersion in oil; 30mg) and was then stirred with methanesulphonyl chloride (1M solution in dry DMF; 1ml) for 45 min. Deprotection, work-up and purification gave the free base of the title compound (60mg) as a solid. Maleate formation gave the title compound - (57mg), m.p. 152-155°.

| | | | |
|---|---|---|---|
| Analysis Found: | C,53.2; | H,4.7; | N,11.7; |
| $C_{17}H_{18}N_4O_3S.C_4H_4O_4$ requires | C,53.2; | H,4.7; | N,11.8%. |

Example 11

### 2,3,4,5-Tetrahydro-2-[(5-methyl-1H-imidazol-4-yl)methyl]-5-(2-propynyl)-1H-pyrido[4,3-b]indol-1-one maleate

A suspension of 2,3,4,5-tetrahydro-2-[[5-methyl-1-(triphenylmethyl)-1H-imidazol-4-yl]methyl]-1H-pyrido[4,3-b]indol-1-one (522mg) and potassium carbonate (276mg) in dry acetone (75mℓ) was treated with propargyl bromide (1M solution in acetone; 2ml) and the mixture was heated at reflux overnight. After cooling, excess acetone was removed in vacuo to give an oil which was partitioned between water (100mℓ) and ethyl acetate (100mℓ). The aqueous phase was washed with ethyl acetate (50mℓ) and the combined organic extracts were concentrated in vacuo. Deprotection, work-up and purification gave the free base of the title compound (100mg) as a solid. Maleate formation gave the title compound (89mg), m.p. 202-205°, t.l.c. (System A, 100:8:1) Rf 0.29.

Example 12

### 2,3,4,5-Tetrahydro-2-[(5-methyl-1H-imidazol-4-yl)methyl]-5-(2-propenyl)-1H-pyrido[4,3-b]indol-1-one maleate

2,3,4,5-Tetrahydro-2-[[5-methyl-1-(triphenylmethyl)-1H-imidazol-4-yl]methyl]-1H-pyrido[4,3-b]indol-1-one (1.0g) was treated with sodium hydride (60% dispersion in oil; 114mg) and was then stirred with allyl bromide (460mg) for 1 h. Deprotection, work-up and purification gave the free base of the title compound

(380mg) as a solid. Maleate formation gave the title compound (160mg), t.l.c. (System A, 100:8:1) Rf 0.3.

| | | | |
|---|---|---|---|
| Analysis Found: | C,63.2; | H,5.5; | N,12.5; |
| $C_{19}H_{20}N_4O.C_4H_4O_4$ requires | C,63.3; | H,5.5; | N,12.8%. |

Example 13

5-Cyclopentyl-2,3,4,5-tetrahydro-2-[(5-methyl-1H-imidazol-4-yl)methyl]-1H-pyrido[4,3-b]indol-1-one maleate

A solution of 2,3,4,5-tetrahydro-2-[[5-methyl-1-(triphenylmethyl)-1H-imidazol-4-yl]methyl]-1H-pyrido[4,3-b]-indol-1-one (523mg) in dry DMF (30ml) was treated with sodium hydride (60% dispersion in oil; 46mg) and stirred for 15 min. at 21° under nitrogen. Cyclopentyl bromide (298mg) was then added dropwise, and the mixture was stirred for 1h and then heated at reflux for 4h. The solution was left at 21° for 2 days, and then treated with a mixture of acetic acid (7mℓ), water (7mℓ) and THF (8mℓ). The resulting solution was heated at reflux for 4h, then basified with 2N sodium hydroxide and extracted with dichloromethane (3x25mℓ). The combined extracts were washed with water (2x50mℓ), concentrated in vacuo and purified by FCC eluting with System A (100:8:1) to give the free base of the title compound (42mg) as a solid. Maleate formation gave the title compound (38mg), m.p. 180° (decomp.), t.l.c. (System A, 100:8:1) Rf 0.3.

Example 14

2,3,4,5-Tetrahydro-2-[(5-methyl-1H-imidazol-4-yl)methyl]-5-propyl-1H-pyrido[4,3-b]indol-1-one maleate

A solution of 2,3,4,5-tetrahydro-2-[(5-methyl-1H-imidazol-4-yl)methyl]-5-(2-propenyl)-1H-pyrido[4,3-b]indol-1-one (248mg) in a mixture of ethanol (20mℓ) and 2N hydrochloric acid (0.5mℓ) was hydrogenated at room temperature and atmospheric pressure over a pre-reduced 10% palladium oxide on carbon catalyst (50% aqueous paste; 50mg). The mixture was filtered and evaporated in vacuo. The residue was basified with 2N sodium hydroxide (10mℓ) and extracted with dichloromethane (3x20mℓ). The combined organic extracts were washed with water (30mℓ) and evaporated to give the free base of the title compound (258mg) as a solid. Maleate formation gave the title compound (345mg), t.l.c. (System A, 100:8:1) Rf 0.4.
Water Analysis Found 1.13% w/w ≡ 0.28mol $H_2O$.

| | | | |
|---|---|---|---|
| Analysis Found: | C,62.1; | H,5.9; | N,12.5; |
| $C_{19}H_{22}N_4O.C_4H_4O_4$ 0.28$H_2O$ requires | C,62.2; | H,6.0; | N,12.6%. |

Example 15

2,3,4,5-Tetrahydro-2-[(5-methyl-1H-imidazol-4-yl)methyl]-1H-pyrido-[4,3-b]indol-1-one maleate

A suspension of 2,3,4,5-tetrahydro-2-[(5-methyl-1H-imidazol-4-yl)methyl]-5-[phenyl(methoxymethyl)]-1H-pyrido[4,3-b]indol-1-one (400mg) in ethanol (20mℓ) and glacial acetic acid (5mℓ) was hydrogenated overnight at room temperature and atmospheric pressure over a pre-reduced 10% palladium oxide on

carbon catalyst (50% aqueous paste; 100mg). The reaction mixture was filtered and the residue was washed with ethanol (100mℓ). The filtrate was concentrated in vacuo to give an oil, to which was added 2N sodium hydroxide (50mℓ). The resulting suspension was extracted with dichloromethane (2x50mℓ) and the combined, dried organic extracts were evaporated to give a solid. This was purified by FCC eluting with System A (75:8:1) to give the free base of the title compound as a solid (240mg) which was then dissolved in dry methanol (50mℓ). Maleate formation gave the title compound (261mg), t.l.c. (System A, 75:8:1) Rf 0.2.

| | | | |
|---|---|---|---|
| Analysis Found: | C,60.3; | H,5.2; | N,13.8; |
| $C_{16}H_{16}N_4O.C_4H_4O_4$ requires | C,60.6; | H,5.1; | N,14.1%. |

Example 16

## 2,3,4,5-Tetrahydro-5-methyl-2-[(1,5-dimethyl-1H-imidazol-4-yl)methyl]-1H-pyrido[4,3-b]indol-1-one maleate

Sodium hydride (73% dispersion in oil; 40mg) was added to a stirred suspension of 2,3,4,5-tetrahydro-5-methyl-2-[(5-methyl-1H-imidazol-4-yl)methyl]-1H-pyrido[4,3-b]indol-1-one (300mg) in dry DMF (3mℓ) under nitrogen. After 30 min. the suspension was cooled to 0° and iodomethane (0.076mℓ) was added dropwise. The mixture was allowed to reach room temperature, stirred for 1.5h, then poured into water (30mℓ) and extracted with dichloromethane (3x15mℓ). The combined, dried organic extracts were evaporated to give an oil (ca. 545mg) which was purified by FCC eluting with System A (200:8:1) to give a solid (95mg). A portion of this material (90mg) was dissolved in absolute ethanol (3mℓ) and treated with a solution of maleic acid (35mg) in absolute ethanol (1mℓ). The solvent was removed in vacuo and the residue was triturated with dry ether (3x5mℓ) to give the title compound (122mg), m.p. 178-180°.

| | | | |
|---|---|---|---|
| Analysis Found: | C,62.1; | H,5.7; | N,13.1; |
| $C_{18}H_{20}N_4O.C_4H_4O_4$ requires | C,62.3; | H,5.7; | N,13.2%. |

Example 17

## 2,3,4,5-Tetrahydro-2-[(1H-imidazol-4-yl)methyl]-5-methyl-1H-pyrido[4,3-b]indol-1-one dimaleate

A solution of 2,3,4,5-tetrahydro-5-methyl-2-[[1-(triphenylmethyl)-1H-imidazol-4-yl]methyl]-1H-pyrido[4,3-b]-indol-1-one (0.22g) in a mixture of acetic acid, THF and water (1:1:1; 10mℓ) was heated on a steam bath for 30 min. The suspension so obtained was diluted with 1M hydrochloric acid (20mℓ) and washed with ethyl acetate (3x20mℓ). The acidic aqueous phase was basified with solid sodium carbonate and extracted with dichloromethane:methanol (9:1; 3x20mℓ). The combined, dried organic extracts were evaporated to give a foam which was dissolved in methanol (5mℓ) and treated with a solution of maleic acid (0.15g) in methanol (5mℓ). The clear solution was evaporated to give a gum which on trituration with ether afforded the title compound (0.17g) as a solid, m.p. 117-118°.

| | | | |
|---|---|---|---|
| Analysis Found: | C,56.1; | H,4.3; | N,10.5; |
| $C_{16}H_{16}N_4O.2C_4H_4O_4$ requires | C,56.2; | H,4.7; | N,10.9%. |

Example 18

## 2,3,4,5-Tetrahydro-5-methyl-2-[(5-methyl-1H-imidazol-4-yl)methyl]-1H-pyrido[4,3-b]indol-1-one hydrochloride

2,3,4,5-Tetrahydro-5-methyl-2-[(5-methyl-1H-imidazol-4-yl)methyl]-1H-pyrido[4,3-b]indol-1-one (1.00g) was suspended in ethanol (40ml) and concentrated hydrochloric acid (1.00ml) was added. The mixture was warmed to 40° and charcoal (0.25g) was added. The resulting suspension was stirred and warmed for 5 min. and then filtered. The filtrate was evaporated in vacuo to ca. 20ml and was allowed to cool to 20°. Ether (40ml) was added with stirring over 5 min., and the mixture was stored at 4° overnight. The resulting precipitate was filtered off, washed with ether (2x10ml), dried in vacuo at room temperature for 2h and then at 70° for 7 h to give the title compound (0.95g), m.p. 288-291°.

| | | | | |
|---|---|---|---|---|
| Analysis Found: | C,61.4; | H,5.8; | N,16.7; | Cl, 10.7; |
| $C_{17}H_{18}N_4O.HCl$ requires | C,61.7; | H,5.8; | N,16.9; | Cl, 10.7%. |

Example 19

## 2,3,4,5-Tetrahydro-5-methyl-2-[(5-methyl-1H-imidazol-4-yl)methyl]-1H-pyrido[4,3-b]indol-1-one sulphate

2,3,4,5-Tetrahydro-5-methyl-2-[(5-methyl-1H-imidazol-4-yl)methyl]-1H-pyrido[4,3-b]indol-1-one (0.81g) was suspended in absolute ethanol (6ml) and was warmed at 50° with concentrated sulphuric acid (0.15ml). More ethanol (4ml) was added and the mixture was stirred with charcoal (0.1g). The suspension was then filtered and the collected solid was washed with ethanol (ca. 3ml). The resulting filtrate was stirred for ca. 1 h at room temperature, tert-butyl methyl ether (10ml) was added slowly and the mixture was stirred for 20 min. The precipitate was filtered off, washed with ethanol:tert-butyl methyl ether (1:1;6ml), then with tert-butyl methyl ether (6ml), and dried in vacuo at 40° for 4 days to give the title compound (0.4g), m.p. 205-209°.

| | | | | |
|---|---|---|---|---|
| Analysis Found: | C,49.5; | H,5.6; | N,13.5; | S,8.4; |
| $C_{17}H_{18}N_4O.\ 1.1H_2SO_4$ requires | C,49.9; | H,5.4; | N,13.3; | S,8.4%. |

Example 20

## 2,3,4,5-Tetrahydro-5-methyl-2-[(5-methyl-1H-imidazol-4-yl)methyl]-1H-pyrido[4,3-b]indol-1-one

A suspension of 2,3,4,5-tetrahydro-5-methyl-1H-pyrido[4,3-b]indol-1-one (400mg) in dry DME (50mℓ) was treated with sodium hydride (60% dispersion in oil; 100mg), and the mixture was stirred at 60° under nitrogen for 6h. 4-(Chloromethyl)-5-methyl-1-(triphenylmethyl)-1H-imidazole (474mg) was added and the reaction mixture was stirred at 60° under nitrogen overnight. 2N Hydrochloric acid (10mℓ) and water (10mℓ) were then added, and the mixture was heated at reflux for 6h. After cooling, the mixture was basified with 2N sodium hydroxide and the resulting mixture was extracted with ethyl acetate (2x50mℓ). The combined, dried organic extracts were concentrated onto FCC silica and purified by FCC eluting with

System A (150:8:1) to give the title compound (352mg) as a solid, t.l.c. (System A, 100:8:1) Rf 0.28. $^1$H-N.m.r.: δ 2.2 (3H,s), 3.04 (2H,t), 3.62 (2H,t), 3.72 (3H,s), 4.53 (2H,s), 7.1-7.28 (2H,m), 7.43 (1H,s), 7.47-7.55 (1H,dd), 7.94-8.03 (1H,dd).

Example 21

## 2,3,4,5-Tetrahydro-5-methyl-2-[(5-methyl-1H-imidazol-4-yl)methyl]-1H-pyrido[4,3-b]indol-1-one

A mixture of 2,5-dihydro-5-methyl-2-[[(5-methyl-1H-imidazol-4-yl)methyl]-1H-pyrido[4,3-b]indol-1-one (50mg) and 10% palladium oxide on carbon catalyst (50% aqueous paste; 25mg) in absolute ethanol (10mℓ) was heated at 80° in a hydrogen atmosphere at 80 p.s.i. for 24h. The suspension was filtered and the filtrate was evaporated to give an oil (49mg) which was purified by short path column chromatography on silica gel (Merck 7739) eluting with System A (150:10:1) to give the title compound (8mg) as a solid, t.l.c. (System A, 150:10:1) Rf 0.36. The $^1$H-n.m.r. data obtained for this material were consistent with those obtained for the product of Example 20.

Example 22

## 2,3,4,5-Tetrahydro-5-methyl-2-[(5-methyl-1H-imidazol-4-yl)methyl]-1H-pyrido[4,3-b]indol-1-one

A solution of 2,3,4,5-tetrahydro-2-[[5-methyl-1-(triphenylmethyl)-1H-imidazol-4-yl]methyl]-1H-pyrido[4,3-b]-indol-1-one (261mg) in dry DMF (25mℓ) was treated with sodium hydride (60% dispersion in oil; 30mg) and the mixture was stirred at room temperature under nitrogen for 15 min. Iodomethane (0.5M solution in DMF; 2mℓ) was then added and stirring was continued for a further 15 min. The reaction mixture was then poured into water (100mℓ) and the resulting suspension was extracted with ethyl acetate (2 x 50mℓ). The combined organic extracts were washed with water (2 x 100mℓ), dried and concentrated to give a solid. This was dissolved in a mixture of water (10mℓ), THF (10mℓ) and glacial acetic acid (10mℓ) and heated at reflux for 2 h. After cooling, residual THF was removed in vacuo and the remaining solution was basified (to pH14) by addition of 2N sodium hydroxide. The resulting suspension was extracted with ethyl acetate (2 x 50mℓ) and the combined, dried organic extracts wre concentrated onto silica (Merck 7385). FCC eluting with System A (100:8:1) gave the title compound (81mg) as a solid. The $^1$H-n.m.r. and t.l.c. data obtained for this material were consistent with those obtained for the product of Example 20.

Example 23

## 2,3,4,5-Tetrahydro-5-methyl-2-[(5-methyl-1H-imidazol-4-yl)methyl]-1H-pyrido[4,3-b]indol-1-one

5,6-Dihydro-1-[(5-methyl-1H-imidazol-4-yl)methyl]-4-(2-methyl-2-phenylhydrazino)-2(1H)-pyridinone (20.0mg) was dissolved in 98% sulphuric acid (1mℓ) and the solution was stirred at 25° for 5 min. The mixture was cautiously poured into 8% aqueous sodium bicarbonate solution (60mℓ) and extracted with 10% methanol:dichloromethane (2x60mℓ). The combined, dried organic extracts were evaporated in vacuo to leave an oil which was purified by FCC eluting with System A (100:8:1) to give the title compound (13.5mg) as a solid. The $^1$H-n.m.r. and t.l.c. data obtained for this material were consistent with those obtained for the product of Example 20.

Example 24

2,3,4,5-Tetrahydro-5-methyl-2-[(5-methyl-1H-imidazol-4-yl)methyl]-1H-pyrido[4,3-b]indol-1-one

A solution of N,N,5-trimethyl-4-[[1,2,3,6-tetrahydro-4-[(2-iodophenyl) methylamino]-6-oxo-1-pyridinyl]methyl]-1H-imidazole-1-sulphonamide (264mg) in a mixture of dioxane and acetonitrile (2:1; 200mℓ) containing triethylamine (2mℓ) was irradiated in a pyrex immersion well with a medium pressure 125W mercury lamp at room temperature for 24h. The reaction mixture was then concentrated onto FCC silica and purified by FCC eluting with System A (150:8:1) to give the title compound (87mg) as a solid. The $^1$H-n.m.r. and t.l.c. data obtained for this material were consistent with those obtained for the product of Example 20.

Example 25

2,3,4,5-Tetrahydro-5-methyl-2-[(5-methyl-1H-imidazol-4-yl)methyl]-1H-pyrido[4,3-b]indol-1-one

A solution of N,N,5-trimethyl-4-[(2,3,4,5-tetrahydro-5-methyl-1-oxo-1H-pyrido[4,3-b]indol-2-yl)methyl]-1H-imidazole-1-sulphonamide (86mg) in 2N hydrochloric acid (10mℓ) and absolute ethanol (2mℓ) was heated at 100-110° for 4h. The reaction mixture was then cooled and 2N sodium hydroxide (50mℓ) was added. The resulting solution was extracted with dichloromethane (2x50mℓ) and the combined, dried organic extracts were concentrated onto FCC silica and purified by FCC eluting with System A (100:8:1) to give a solid (36mg). This was taken up in hot ethyl acetate and purified by slow evaporation to give the title compound - (12mg). The $^1$H-n.m.r. and t.l.c. data obtained for this material were consistent with those obtained for the product of Example 20.

Example 26

2,3,4,5-Tetrahydro-5-methyl-2-[(5-methyl-1H-imidazol-4-yl)methyl]-1H-pyrido[4,3-b]indol-1-one

A solution of N,N,5-trimethyl-4-[(2,3,4,5-tetrahydro-5-methyl-1-oxo-1H-pyrido[4,3-b]indol-2-yl)methyl]-1H-imidazole-1-sulphonamide (401mg) in a mixture of dioxane (150mℓ) and acetonitrile (150mℓ) containing triethylamine (1mℓ) was irradiated at room temperature with a medium pressure mercury lamp for 24h. The reaction mixture was then concentrated in vacuo onto FCC silica and purified by FCC eluting with System A (100:8:1) to give the title compound (203mg) as a solid. The $^1$H-n.m.r. and t.l.c. data obtained for this material were consistent with those obtained for the product of Example 20.

Example 27

2,3,4,5-Tetrahydro-5-methyl-2-[(5-methyl-1H-imidazol-4-yl)methyl]-1H-pyrido[4,3-b]indol-1-one

A solution of phenylmethyl 5-methyl-4-[(2,3,4,5-tetrahydro-5-methyl-1-oxo-1H-pyrido[4,3-b]indol-2-yl)-methyl]-1H-imidazole-1-carboxylate (134mg) in a mixture of absolute ethanol and 2N hydrochloric acid (2:1; 30mℓ) was heated on a steam bath for 15 min. After cooling, the solution was concentrated in vacuo to ca.

20mℓ and diluted with water (40mℓ). The mixture was then washed with ethyl acetate (2x40mℓ) and the acidic aqueous layer was basified with potassium carbonate solution. The solution was then extracted with ethyl acetate (3x50mℓ) and the combined, dried organic extracts, were concentrated onto FCC silica and purified by FCC eluting with System A (150:8:1) to give a solid. This was dissolved in hot methanol and triturated with ether to give the title compound (69mg). The $^1$H-n.m.r. and t.l.c. data obtained for this material were consistent with those obtained for the product of Example 20.

Example 28

2,3,4,5-Tetrahydro-5-methyl-2-[(5-methyl-1H-imidazol-4-yl)methyl]-1H-pyrido[4,3-b]indol-1-one

A solution of 2,3,4,5-tetrahydro-2-[[1-(methoxymethyl)-5-methyl-1H-imidazol-4-yl]methyl]-5-methyl-1H-pyrido[4,3-b]indol-1-one and 2,3,4,5-tetrahydro-2-[[1-(methoxymethyl)-4-methyl-1H-imidazol-5-yl]methyl]-5-methyl-1H-pyrido[4,3-b]indol-1-one (34mg) in 49% hydrobromic acid (2mℓ) was heated on a steam bath for ca. 3h. After cooling, the reaction mixture was basified by addition of potassium carbonate solution and extracted with ethyl acetate (3x50mℓ). The combined, dried organic extracts were concentrated in vacuo to give the title compound (6mg) as a solid. The $^1$H-n.m.r. and t.l.c. data obtained for this material were consistent with those obtained for the product of Example 20.

Example 29

2,3,4,5-Tetrahydro-5-methyl-2-[(5-methyl-1H-imidazol-4-yl)methyl]-1H-pyrido[4,3-b]indol-1-one

A mixture of 2,3,4,5-tetrahydro-5-methyl-2-[(4-methyloxazol-5-yl)methyl]-1H-pyrido[4,3-b]indol-1-one (100mg) in formamide (20mℓ) was heated at 180° for 24h. The mixture was then cooled, diluted with water (100mℓ) and extracted with dichloromethane (3x100mℓ). The combined organic extracts were concentrated in vacuo and the residue was purified by FCC eluting with System A (100:8:1) to give the title compound - (40mg) as a solid. The $^1$H-n.m.r. and t.l.c. data for this material were consistent with those obtained for the product of Example 20.

Example 30

2,3,4,5-Tetrahydro-5-methyl-2-[(5-methyl-1H-imidazol-4-yl)methyl]-1H-pyrido[4,3-b]indol-1-one

A solution of 4-[[[(1-methyl-1H-indol-2-yl)ethyl]amino]methyl]-N,N,5-trimethyl-1H-imidazole-1-sulphonamide (140mg) in dry dichloromethane (15mℓ) was cooled to 5° and the mixture was stirred under nitrogen while phosgene (12% w/w solution in toluene; 1mℓ) was added dropwise. The reaction mixture was stirred at room temperature for 2h, aluminium trichloride (60mg) was powdered and added, and stirring was continued overnight. After this time methanol (1mℓ) was added and the reaction mixture was adsorbed onto FCC silica and purified by FCC eluting with System A (150:8:1) to give the protected derivative of the title compound (42mg), as a solid, identical (by t.l.c. and m.p.) to the product of Intermediate 27. Deprotection as described in either of Examples 25 or 26 gives the title compound.

Example 31

## 2,3,4,5-Tetrahydro-5-methyl-2-[(5-methyl-1H-imidazol-4-yl)methyl]-1H-pyrido[4,3-b]indol-1-one

A mixture of 4-[[[(3-iodo-1-methyl-1H-indol-2-yl)ethyl]amino]methyl]-N,N,5-trimethyl-1H-imidazole-1-sulphonamide (70mg), triphenylphosphine (52mg) and palladium acetate (22mg) in tri-n-butylamine (5mℓ) and dry THF (1mℓ) was heated under an atmosphere of carbon monoxide at 120° for 1h. After cooling the reaction mixture was poured into 2N hydrochloric acid (50mℓ) and the resulting mixture was extracted with ethyl acetate (2x50mℓ; discarded). The acidic solution was then basified with 2N potassium carbonate and the resulting basic suspension was extracted with ethyl acetate (2x50mℓ). The combined, dried organic extracts were concentrated in vacuo to give an oil and residual tri-n-butylamine was removed by distillation to leave a solid. This was adsorbed onto FCC silica and purified by FCC eluting with System A (150:8:1) to give the protected derivative of the title compound (21mg) as a solid, identical (by t.l.c. and m.p.) to the product of Intermediate 27. Deprotection as described in either of Examples 25 or 26 gives the title compound.

Example 32

## 2,3,4,5-Tetrahydro-5-methyl-2-[(5-methyl-1H-imidazol-4-yl)methyl]-1H-pyrido[4,3-b]indol-1-one

5,6-Dihydro-1-[(5-methyl-1H-imidazol-4-yl)methyl]-4-(2-methyl-2-phenylhydrazino)-2(1H)-pyridinone (60mg) was dissolved in glacial acetic acid (4mℓ). Anhydrous zinc chloride (600mg) was added, and the mixture was heated at 85° for 1.5h. The cooled mixture was poured into 8% aqueous sodium bicarbonate solution (100mℓ) and extracted with ethyl acetate:methanol (10:1) (2x100mℓ). The combined, dried organic extracts were evaporated in vacuo to leave a solid which was purified by FCC eluting with System A (100:8:1) to give the title compound (26mg). The $^1$H-n.m.r. and t.l.c. data obtained for this material were consistent with those obtained for the product of Example 20.

The following examples illustrate pharmaceutical formulations according to the invention, containing 2,3,4,5-tetrahydro-5-methyl-2-[(5-methyl-1H-imidazol-1-yl)methyl]-1H-pyrido[4,3-b]indol-1-one as the active ingredient. Physiologically acceptable salts and/or solvates of this compound, and other compounds of formula (I) and their physiologically acceptable salts and/or solvates may be formulated in a similar manner.

TABLETS FOR ORAL ADMINISTRATION

Tablets may be prepared by the normal methods such as direct compression or wet granulation.

The tablets may be film coated with suitable film forming materials, such as hydroxypropyl methylcellulose, using standard techniques. Alternatively the tablets may be sugar coated.

Direct Compression

| Tablet | mg/tablet |
|---|---|
| Active Ingredient | 0.50 |
| Calcium Hydrogen Phosphate BP* | 87.25 |
| Croscarmellose Sodium NF | 1.80 |
| Magnesium Stearate BP | 0.45 |
| Compression weight | 90.00 |

* of a grade suitable for direct compression.

The active ingredient is passed through a 60 mesh sieve, blended with the calcium hydrogen phosphate, croscarmellose sodium and magnesium stearate. The resultant mix is compressed into tablets using a Manesty F3 tablet machine fitted with 5.5mm, flat bevelled edge punches.

| Sub-Lingual Tablet | mg/tablet |
|---|---|
| Active Ingredient | 0.5 |
| Compressible Sugar NF | 64.5 |
| Magnesium Stearate BP | 0.5 |
| Compression Weight | 65.0 |

The active ingredient is sieved through a suitable sieve, blended with the excipients and compressed using suitable punches. Tablets of other strengths may be prepared by altering either the ratio of active ingredient to excipients or the compression weight and using punches to suit.

Wet Granulation

| Conventional Tablet | mg/tablet |
|---|---|
| Active Ingredient | 0.5 |
| Lactose BP | 153.5 |
| Starch BP | 30.0 |
| Pregelatinised Maize Starch BP | 15.0 |
| Magnesium Stearate BP | 1.5 |
| Compression Weight | 200.0 |

The active ingredient is sieved through a suitable sieve and blended with lactose, starch and pregelatinised maize starch. Suitable volumes of purified water are added and the powders are granulated. After drying, the granules are screened and blended with the magnesium stearate. The granules are then compressed into tablets using 7mm diameter punches.

Tablets of other strengths may be prepared by altering the ratio of active ingredient to lactose or the compression weight and using punches to suit.

| Sub-Lingual Tablet | mg/tablet |
|---|---|
| Active Ingredient | 0.5 |
| Mannitol BP | 58.5 |
| Hydroxypropylmethylcellulose | 5.0 |
| Magnesium Stearate BP | 1.0 |
| Compression Weight | 65.0 |

The active ingredient is sieved through a suitable sieve and blended with the mannitol and hydroxypropylmethylcellulose. Suitable volumes of purified water are added and the powders are granulated. After

drying, the granules are screened and blended into tablets using suitable punches.

Tablets of other strengths may be prepared by altering the ratio of active ingredient to mannitol or the compression weight and punches to suit.

| CAPSULES | mg/capsule |
|---|---|
| Active Ingredient | 0.5 |
| * Starch 1500 | 98.5 |
| Magnesium Stearate BP | 1.0 |
| Fill Weight | 100.0 |

* a form of directly compressible starch.

The active ingredient is sieved and blended with the excipients. The mix is filled into size No. 2 hard gelatin capsules using suitabled machinery. Other doses may be prepared by altering the fill weight an if necessary changing the capsule size to suit.

SYRUP

This may be either a sucrose or sucrose free presentation.

| A. Sucrose Syrup | | mg/5ml dose |
|---|---|---|
| Active Ingredient | | 0.5 |
| Sucrose BP | | 2750.0 |
| Glycerine BP | | 500.0 |
| Buffer )<br>Flavour )<br>Colour )<br>Preservative ) | | as required |
| Purified Water BP | to | 5.0ml |

The active ingredient, buffer, flavour, colour and preservative are dissolved in some of the water and the glycerine is added. The remainder of the water is heated to dissolve the sucrose and is then cooled. The two solutions are combined, adjusted to volume and mixed. The syrup is clarified by filtration.

| B. Sucrose-Free | | mg/5ml dose |
|---|---|---|
| Active Ingredient | | 0.5 |
| Hydroxypropylmethylcellulose USP (viscosity type 4000) | | 22.5 |
| Buffer )<br>Flavour )<br>Colour )<br>Preservative )<br>Sweetener ) | | as required |
| Purified Water BP | to | 5.0ml |

The hydroxypropylmethylcellulose is dispersed in hot water, cooled and then mixed with an aqueous solution containing the active ingredient and the other components of the formulation. The resultant solution is adjusted to volume and mixed. The syrup is clarified by filtration.

| INJECTION FOR INTRAVENOUS ADMINISTRATION | | |
|---|---|---|
| | mg/mℓ | |
| Active ingredient<br>Sodium Chloride BP<br>Water for Injection BP to | 0.05<br>as required<br>1.0mℓ | 0.5<br>as required<br>1.0mℓ |

Sodium chloride may be added to adjust the tonicity of the solution and the pH may be adjusted, using acid or alkali, to that of optimum stability and/or facilitate solution of the active ingredient. Alternatively, suitable buffer salts may be used.

The solution is prepared, clarified and filled into appropriate size ampoules sealed by fusion of the glass. The injection is sterilised by heating in an autoclave using one of the acceptable cycles. Alternatively, the solution may be sterilised by filtration and filled into sterile ampoules under aseptic conditions. The solution may be packed under an inert atmosphere of nitrogen or other suitable gas.

| METERED DOSE PRESSURISED AEROSOL | | |
|---|---|---|
| Suspension Aerosol | mg/metered dose | Per can |
| Active Ingredient micronised | 0.050 | 12.0mg |
| Oleic Acid BP | 0.020 | 4.80mg |
| Trichlorofluoromethane BP | 23.64 | 5.67g |
| Dichlorodifluoromethane BP | 61.25 | 14.70g |

The active ingredient is micronised in a fluid energy mill to a fine particle size range. The oleic acid is mixed with the trichlorofluoromethane at a temperature of 10-15°C and the micronised drug is mixed into the solution with a high shear mixer. The suspension is metered into aluminium aerosol cans and suitable metering valves, delivering 85mg of suspension are crimped onto the cans and the dichlorodifluoromethane is pressure filled into the cans through the valves.

| Solution Aerosol | | |
|---|---|---|
| | mg/metered dose | Per can |
| Active Ingredient | 0.05 | 12.0mg |
| Ethanol BP | 7.500 | 1.80g |
| Trichlorofluoromethane BP | 18.875 | 4.53g |
| Dichlorodifluoromethane BP | 48.525 | 11.65g |

Oleic acid BP, on a suitable surfactant e.g. Span 85 (sorbitan trioleate) may also be included).

The active ingredient is dissolved in the ethanol together with the oleic acid or surfactant if used. The alcoholic solution is metered into suitable aerosol containers followed by the trichlorofluoromethane. Suitable metering valves are crimped onto the containers and dichlorodifluoromethane is pressure filled into them through the valves.

| Inhalation Cartridges | |
|---|---|
| | mg/cartridge |
| Active Ingredient (micronised) | 0.05 |
| Lactose BP to | 25.00 |

The active ingredient is micronised in a fluid energy mill to a fine particle size range prior to blending with normal tabletting grade lactose in a high energy mixer. The powder blend is filled into No. 3 hard gelatin capsules on a suitable encapsulating machine. The contents of the cartridges are administered using a powder inhaler.

| SUPPOSITORY | |
|---|---|
| Active Ingredient<br>* Witepsol H15 to | 0.5mg<br>1.0g |

* Witepsol H15 is a proprietary grade of Adeps Solidus Ph. Eur.

A suspension of the active ingredient is prepared in the molten Witepsol and filled, using suitable machinery, into 1g size suppository moulds.

**Claims**

**Claims for the following Contracting States : AT, BE, CH, DE, FR, GB, IT, LI, LU, NL, SE**

**1.** Compounds of the general formula (I)

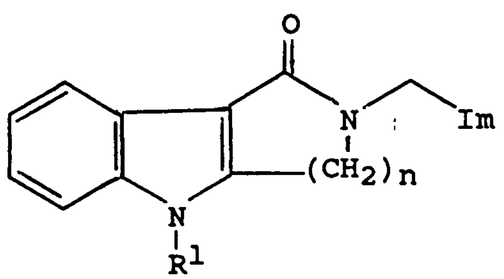

(I)

wherein Im represents an imidazolyl group of the formula:

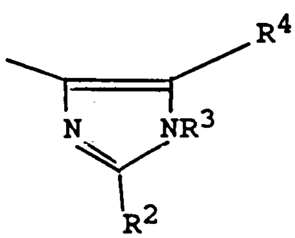

or

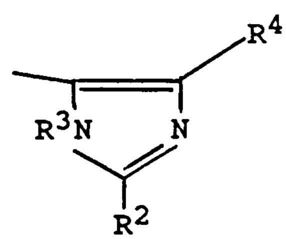

and $R^1$ represents a hydrogen atom or a group selected from $C_{1-6}$alkyl, $C_{3-6}$alkenyl, $C_{3-10}$alkynyl, $C_{3-7}$cycloalkyl, $C_{3-7}$cycloalkyl$C_{1-4}$alkyl, phenyl, phenyl$C_{1-3}$alkyl, phenylmethoxymethyl, phenoxyethyl, phenoxymethyl, $-CO_2R^5$, $-COR^5$, $-CONR^5R^6$ or $-SO_2R^5$ (wherein $R^5$ and $R^6$, which may be the same or different, each represents a hydrogen atom, a $C_{1-6}$alkyl or $C_{3-7}$cycloalkyl group or a phenyl or phenyl$C_{1-4}$alkyl group, in which the phenyl group is optionally substituted by one or more $C_{1-4}$alkyl, $C_{1-4}$alkoxy or hydroxy groups or halogen atoms, with the proviso that $R^5$ does not represent a hydrogen atom when $R^1$ represents a group $-CO_2R^5$ or $-SO_2R^5$);

one of the groups represented by $R^2$, $R^3$ and $R^4$ is a hydrogen atom or a $C_{1-6}$alkyl, $C_{3-7}$cycloalkyl, $C_{3-6}$alkenyl, phenyl or phenyl$C_{1-3}$alkyl group, and each of the other two groups, which may be the same or different, represents a hydrogen atom or a $C_{1-6}$alkyl group;

n represents 2 or 3;

and physiologically acceptable salts and solvates thereof.

**2.** Compounds as claimed in claim 1 in which $R^1$ represents a $C_{1-4}$alkyl, $C_{3-4}$alkynyl, $C_{5-6}$cycloalkyl, $C_{5-6}$cycloalkylmethyl, phenyl$C_{1-2}$alkyl, phenylmethoxymethyl, or N,N-di$C_{1-3}$alkylcarboxamido group.

**3.** Compounds as claimed in claim 1 or 2 in which $R^2$, $R^3$ and $R^4$ each independently represent a hydrogen atom or a $C_{1-3}$alkyl group.

4. Compounds as claimed in claim 1 in which $R^1$ represents a hydrogen atom or a $C_{1-4}$alkyl, $C_{3-4}$alkenyl,$C_{3-4}$alkynyl, $C_{5-6}$cycloalkyl, $C_{5-6}$cycloalkylmethyl, phenyl$C_{1-2}$alkyl, phenylmethoxymethyl, N,N-di$C_{1-3}$alkylcarboxamido or $C_{1-3}$alkylsulphonyl group;
$R^2$ represents a hydrogen atom; and $R^3$ and $R^4$ each represent a hydrogen atom or a $C_{1-3}$alkyl group.

5. Compounds as claimed in claim 1 in which $R^1$ represents a methyl, n-propyl, prop-2-ynyl, cyclopentyl, cyclopentylmethyl, benzyl or N,N-dimethylcarboxamido group;
$R^2$ and $R^3$ each represent a hydrogen atom; and $R^4$ represents a methyl group.

6. Compounds as claimed in claim 4 or 5 in which n represents 2.

7. 2,3,4,5-Tetrahydro-5-methyl-2-[(5-methyl-1H-imidazol-4-yl)methyl]-1H-pyrido[4,3-b]indol-1-one; and physiologically acceptable salts and solvates thereof.

8. 2,3,4,5-Tetrahydro-5-(phenylmethyl)-2-[(5-methyl-1H-imidazol-4-yl)methyl]-1H-pyrido[4,3-b]indol-1-one;
5-cyclopentyl-2,3,4,5-tetrahydro-2-[(5-methyl-1H-imidazol-4-yl)methyl]-1H-pyrido[4,3-b]indol-1-one;
2,3,4,5-tetrahydro-2-[(5-methyl-1H-imidazol-4-yl)methyl]-5-propyl-1H-pyrido[4,3-b]indol-1-one;
5-(cyclopentylmethyl)-2,3,4,5-tetrahydro-2-[(5-methyl-1H-imidazol-4-yl)methyl]-1H-pyrido[4,3-b]indol-1-one;
3,4,5,6-tetrahydro-6-methyl-2-[(5-methyl-1H-imidazol-4-yl)methyl]-azepino[4,3-b]indol-1(2H)-one;
2,3,4,5-tetrahydro-N,N-dimethyl-2-[(5-methyl-1H-imidazol-4-yl)methyl]-1-oxo-5H-pyrido[4,3-b]indole-5-carboxamide;
2,3,4,5-tetrahydro-2-[(5-methyl-1H-imidazol-4-yl)methyl]-5-(2-propynyl)-1H-pyrido[4,3-b]indol-1-one;
and physiologically acceptable salts and solvates thereof.

9. Compounds as claimed in any of claims 1 to 8 wherein the compound of formula (I) is in the form of a hydrochloride, hydrobromide, sulphate, alkylsulphonate, arylsulphonate, phosphate, acetate, citrate, succinate, tartrate, fumarate or maleate salt.

10. The compound claimed in claim 7 in the form of a hydrochloride salt.

11. The compound claimed in claim 7 in the form of a maleate salt.

12. A process for the preparation of compounds of general formula (I) as defined in any of claims 1 to 11 or a physiologically acceptable salt or solvate thereof, which comprises:
(A) alkylating a compound of formula (II)

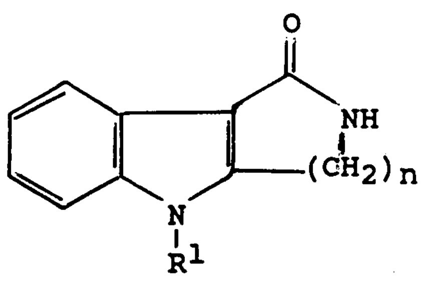

(II)

with a compound of formula (III)

$LCH_2$-Im (III)

or a protected derivative thereof, wherein L represents a leaving atom or group, followed if necessary by removal of any protecting groups present; or
(B) for the preparation of a compound of formula (I) in which n is 2, hydrogenating a compound of formula (IV)

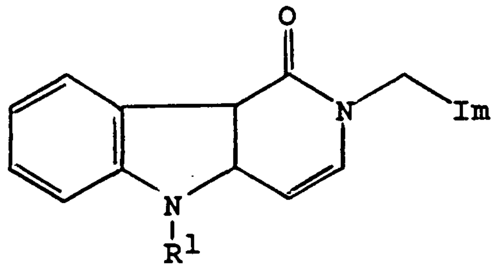

(IV)

or a protected derivative thereof, followed if necessary by removal of any protecting groups present; or

(C) cyclising a compound of formula (V)

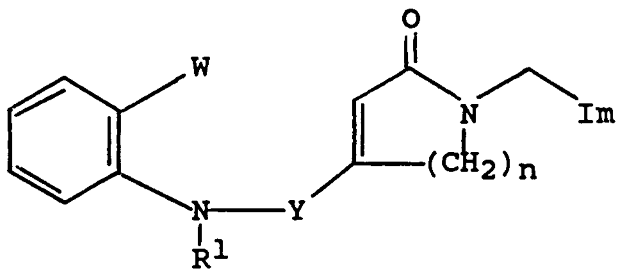

(V)

wherein W represents a hydrogen atom and Y represents the group NH, or W represents a halogen atom and Y represents a bond, or a salt or protected derivative thereof, followed if necessary by removal of any protecting groups present; or

(D) for the preparation of a compound of formula (I) in which $R^3$ represents a hydrogen atom, reacting a compound of formula (VI)

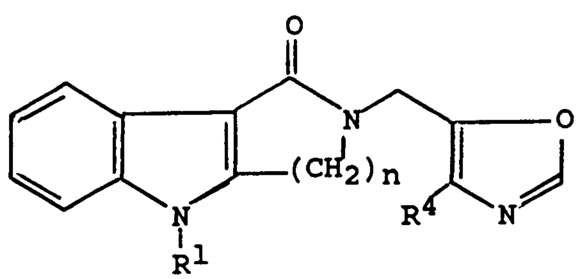

(VI)

or a protected derivative thereof, with formamide, followed if necessary by removal of any protecting groups present; or

(E) reacting a compound of formula (VII)

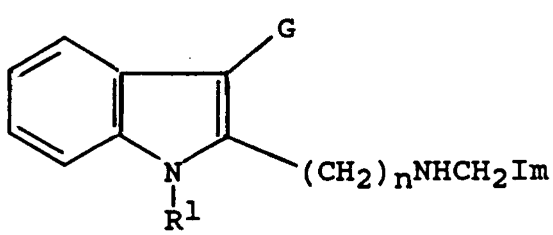

(VII)

where G represents a hydrogen atom, or a protected derivative thereof, with phosgene in the

presence of a Lewis acid; or
where G represents a bromine or iodine atom, or a protected derivative thereof, with carbon monoxide in the presence of a palladium (II) salt; followed if necessary by removal of any protecting groups present; or
(F) converting a compound of general formula (I) into another compound of formula (I) using conventional techniques; or
(G) removing protecting group(s) from a protected form of a compound of formula (I);
and when the compound of formula (I) is obtained as a mixture of enantiomers, optionally resolving the mixture to obtain the desired enantiomer;
and/or where the compound of formula (I) is in the form of a free base, optionally converting the free base into a salt.

**13.** A pharmaceutical composition comprising at least one compound of general formula (I) as defined in claim 1 or a physiologically acceptable salt or solvate thereof together with at least one physiologically acceptable carrier or excipient.

**14.** A pharmaceutical composition as claimed in claim 13 in a form adapted for oral or parenteral administration.

**15.** A pharmaceutical composition as claimed in claim 13 or 14 wherein the active ingredient is 2,3,4,5-tetrahydro-5-methyl-2-[(5-methyl-1H-imidazol-4-yl)methyl]-1H-pyrido[4,3-b]indol-1-one or a physiologically acceptable salt or solvate thereof.

**16.** The use of a compound of the general formula (I) as defined in any of claims 1 to 11 or a physiologically acceptable salt or solvate thereof for the manufacture of a medicament for the treatment of a condition caused by a disturbance of "neuronal" 5-HT function.

**17.** The use of a compound of the general formula (I) as defined in any of claims 1 to 11 or a physiologically acceptable salt or solvate thereof for the manufacture of a medicament for the treatment of nausea and vomiting and/or for the promotion of gastric emptying.

**18.** The use of a compound of the general formula (I) as defined in any of claims 1 to 11 or a physiologically acceptable salt or solvate thereof for the manufacture of a medicament for the treatment of irritable bowel syndrome.

**19.** The use of a compound of the general formula (I) as defined in any of claims 1 to 11 or a physiologically acceptable salt or solvate thereof for the manufacture of a medicament for the treatment of dyspepsia.

**20.** The use of a compound of the general formula (I) as defined in any of claims 1 to 11 or a physiologically acceptable salt or solvate thereof for the manufacture of a medicament for the treatment of reflux oesophagitis.

**Claims for the following Contracting States : GR, ES**

**1.** A process for the preparation of compounds of the general formula (I)

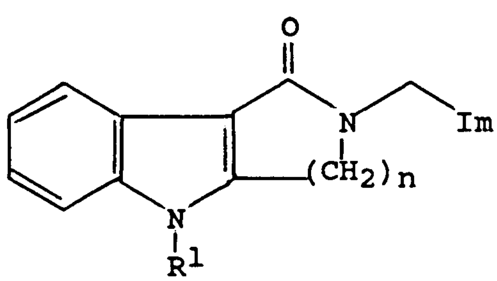

(I)

wherein Im represents an imidazolyl group of the formula:

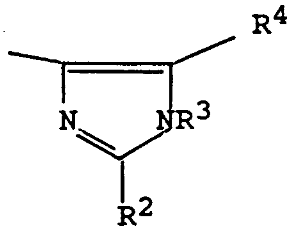

or

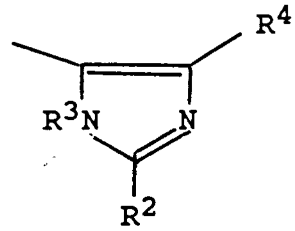

and $R^1$ represents a hydrogen atom or a group selected from $C_{1-6}$alkyl, $C_{3-6}$alkenyl, $C_{3-10}$alkynyl, $C_{3-7}$cycloalkyl, $C_{3-7}$cycloalkyl$C_{1-4}$alkyl, phenyl, phenyl$C_{1-3}$alkyl, phenylmethoxymethyl, phenoxyethyl, phenoxymethyl, $-CO_2R^5$, $-COR^5$, $-CONR^5R^6$ or $-SO_2R^5$ (wherein $R^5$ and $R^6$, which may be the same or different, each represents a hydrogen atom, a $C_{1-6}$alkyl or $C_{3-7}$cycloalkyl group or a phenyl or phenyl$C_{1-4}$alkyl group, in which the phenyl group is optionally substituted by one or more $C_{1-4}$alkyl, $C_{1-4}$alkoxy or hydroxy groups or halogen atoms, with the proviso that $R^5$ does not represent a hydrogen atom when $R^1$ represents a group $-CO_2R^5$ or $-SO_2R^5$);

one of the groups represented by $R^2$, $R^3$ and $R^4$ is a hydrogen atom or a $C_{1-6}$alkyl, $C_{3-7}$cycloalkyl, $C_{3-6}$alkenyl, phenyl or phenyl$C_{1-3}$alkyl group, and each of the other two groups, which may be the same or different, represents a hydrogen atom or a $C_{1-6}$alkyl group;

n represents 2 or 3;

and physiologically acceptable salts and solvates thereof; which comprises:

(A) alkylating a compound of formula (II)

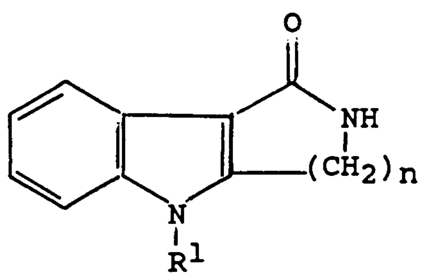

(II)

with a compound of formula (III)

$LCH_2$-Im (III)

or a protected derivative thereof, wherein L represents a leaving atom or group, followed if necessary by removal of any protecting groups present; or

(B) for the preparation of a compound of formula (I) in which n is 2, hydrogenating a compound of formula (IV)

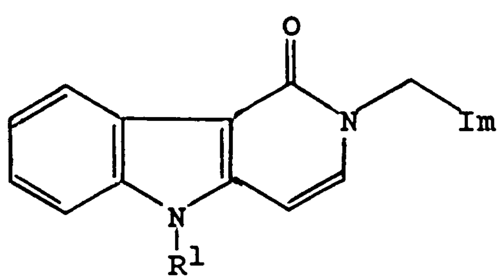

(IV)

or a protected derivative thereof, followed if necessary by removal of any protecting groups present; or

(C) cyclising a compound of formula (V)

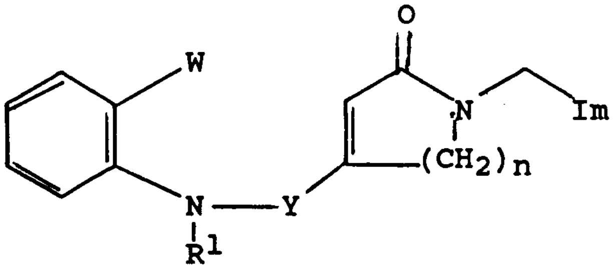

(V)

wherein W represents a hydrogen atom and Y represents the group NH, or W represents a halogen atom and Y represents a bond, or a salt or protected derivative thereof, followed if necessary by removal of any protecting groups present; or

(D) for the preparation of a compound of formula (I) in which $R^3$ represents a hydrogen atom, reacting a compound of formula (VI)

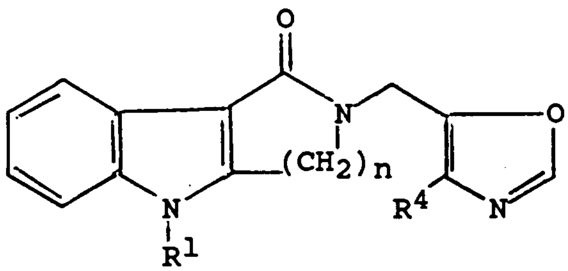

(VI)

or a protected derivative thereof, with formamide, followed if necessary by removal of any protecting groups present; or

(E) reacting a compound of formula (VII)

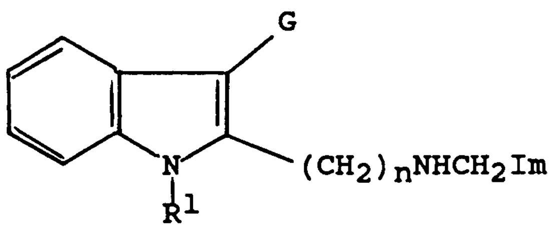

(VII)

where G represents a hydrogen atom, or a protected derivative thereof, with phosgene in the presence of a Lewis acid; or

where G represents a bromine or iodine atom, or a protected derivative thereof, with carbon monoxide in the presence of a palladium (II) salt; followed if necessary by removal of any protecting groups present; or

(F) converting a compound of general formula (I) into another compound of formula (I) using conventional techniques; or

(G) removing protecting group(s) from a protected form of a compound of formula (I);

and when the compound of formula (I) is obtained as a mixture of enantiomers, optionally resolving the mixture to obtain the desired enantiomer;

and/or where the compound of formula (I) is in the form of a free base, optionally converting the free base into a salt.

**2.** A process as claimed in claim 1 for the preparation of compounds in which $R^1$ represents a $C_{1-4}$alkyl, $C_{3-4}$alkynyl, $C_{5-6}$cycloalkyl, $C_{5-6}$cycloalkylmethyl, phenyl$C_{1-2}$alkyl, phenylmethoxymethyl, or N,N-

di$C_{1-3}$alkylcarboxamido group.

3. A process as claimed in claim 1 or 2 for the preparation of compounds in which $R^2$, $R^3$ and $R^4$ each independently represent a hydrogen atom or a $C_{1-3}$alkyl group.

4. A process as claimed in claim 1 for the preparation of compounds in which $R^1$ represents a hydrogen atom or a $C_{1-4}$alkyl, $C_{3-4}$alkenyl, $C_{3-4}$alkynyl, $C_{5-6}$cycloalkyl, $C_{5-6}$cycloalkylmethyl, phenyl$C_{1-2}$alkyl, phenylmethoxymethyl, N,N,-di$C_{1-3}$alkylcarboxamido or $C_{1-3}$alkylsulphonyl group;
$R^2$ represents a hydrogen atom; and $R^3$ and $R^4$ each represent a hydrogen atom or a $C_{1-3}$alkyl group.

5. A process as claimed in claim 1 for the preparation of compounds in which $R^1$ represents a methyl, n-propyl, prop-2-ynyl, cyclopentyl, cyclopentylmethyl, benzyl or N,N,-dimethylcarboxamido group; $R^2$ and $R^3$ each represent a hydrogen atom; and $R^4$ represents a methyl group.

6. A process as claimed in claim 4 or 5 for the preparation of compounds in which n represents 2.

7. A process as claimed in claim 1 for the preparation of the compound 2,3,4,5-tetrahydro-5-methyl-2-[(5-methyl-1H-imidazol-4-yl)methyl]-1H-pyrido[4,3-b]indol-1-one;
and physiologically acceptable salts and solvates thereof.

8. A process as claimed in claim 1 for the preparation of a compound selected from:
2,3,4,5-tetrahydro-5-(phenylmethyl)-2-[(5-methyl-1H-imidazol-4-yl)methyl]-1H-pyrido[4,3-b]indol-1-one;
5-cyclopentyl-2,3,4,5-tetrahydro-2-[(5-methyl-1H-imidazol-4-yl)methyl]-1H-pyrido[4,3-b]indol-1-one;
2,3,4,5-tetrahydro-2-[(5-methyl-1H-imidazol-4-yl)methyl]-5-propyl-1H-pyrido[4,3-b]indol-1-one;
5-(cyclopentylmethyl)-2,3,4,5-tetrahydro-2-[(5-methyl-1H-imidazol-4-yl)methyl]-1H-pyrido[4,3-b]indol-1-one;
3,4,5,6-tetrahydro-6-methyl-2-[(5-methyl-1H-imidazol-4-yl)methyl-azepino[4,3-b]indol-1(2H)-one;
2,3,4,5-tetrahydro-N,N-dimethyl-2-[(5-methyl-1H-imidazol-4-yl)methyl]-1-oxo-5H-pyrido[4,3-b]indole-5-carboxamide;
2,3,4,5-tetrahydro-2-[(5-methyl-1H-imidazol-4-yl)methyl]-5-(2-propynyl)-1H-pyrido[4,3-b]indol-1-one;
and physiologically acceptable salts and solvates thereof.

9. A process as claimed in any of claims 1 to 8 for the preparation of a compound of formula (I) in the form of a hydrochloride, hydrobromide, sulphate, alkylsulphonate, arylsulphonate, phosphate, acetate, citrate, succinate, tartrate, fumarate or maleate salt.

10. A process as claimed in claim 7 for the preparation of the compound in the form of a hydrochloride salt.

11. A process as claimed in claim 7 for the preparation of the compound in the form of a maleate salt.

12. The use of a compound of the general formula (I) as defined in any of claims 1 to 11 or a physiologically acceptable salt or solvate thereof for the manufacture of a medicament for the treatment of a condition caused by a disturbance of "neuronal" 5-HT function.

13. The use of a compound of the general formula (I) as defined in any of claims 1 to 11 or a physiologically acceptable salt or solvate thereof for the manufacture of a medicament for the treatment of nausea and vomiting and/or for the promotion of gastric emptying.

14. The use of a compound of the general formula (I) as defined in any of claims 1 to 11 or a physiologically acceptable salt or solvate thereof for the manufacture of a medicament for the treatment of irritable bowel syndrome.

15. The use of a compound of the general formula (I) as defined in any of claims 1 to 11 or a physiologically acceptable salt or solvate thereof for the manufacture of a medicament for the treatment of dyspepsia.

16. The use of a compound of the general formula (I) as defined in any of claims 1 to 11 or a physiologically acceptable salt or solvate thereof for the manufacture of a medicament for the treatment of reflux oesophagitis.

## Patentansprüche

### Patentansprüche für folgende Vertragsstaaten : AT, BE, CH, DE, FR, GB, IT, LI, LU, NL, SE

1. Verbindungen der allgemeinen Formel (I):

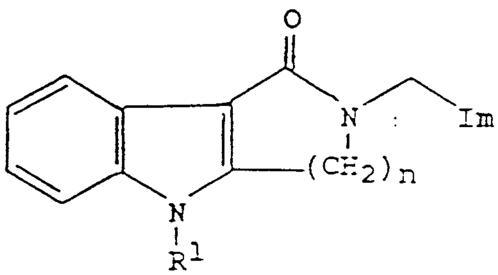

(I)

worin Im für eine Imidazolylgruppe der Formel:

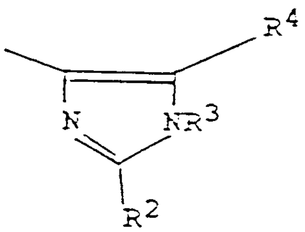

oder

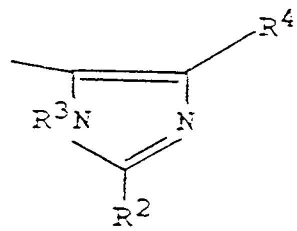

steht;

und $R^1$ für ein Wasserstoffatom oder eine Gruppe, ausgewählt aus $C_{1-6}$-Alkyl, $C_{3-6}$-Alkenyl, $C_{3-10}$-Alkinyl, $C_{3-7}$-Cycloalkyl, $C_{3-7}$-Cyclolalkyl-$C_{1-4}$-alkyl, Phenyl, Phenyl-$C_{1-3}$-alkyl, Phenylmethoxymethyl, Phenoxyethyl, Phenoxymethyl, $-CO_2R^5$, $-COR^5$, $-CONR^5R^6$ oder $-SO_2R^5$, steht (wobei $R^5$ und $R^6$, die gleich oder verschieden sein können, jeweils für ein Wasserstoffatom, eine $C_{1-6}$-Alkyl- oder $C_{3-7}$-Cycloalkylgruppe oder eine Phenyl- oder eine Phenyl-$C_{1-4}$-alkylgruppe stehen, wobei die Phenylgruppe gegebenenfalls durch ein oder mehrere $C_{1-4}$-Alkyl-, $C_{1-4}$-Alkoxy- oder Hydroxygruppen oder Halogenatome substituiert ist, mit der Maßgabe, daß $R^5$ kein Wasserstoffatom ist, wenn $R^1$ für eine Gruppe $-CO_2R^5$ oder $-SO_2R^5$ steht);

eine der Gruppen $R^2$, $R^3$ und $R^4$ ein Wasserstoffatom oder eine $C_{1-6}$-Alkyl-, $C_{3-7}$-Cycloalkyl-, $C_{3-6}$-Alkenyl-, Phenyl- oder oder Phenyl-$C_{1-3}$-alkylgruppe ist und jede der zwei anderen Gruppen, die gleich oder verschieden sein können, ein Wasserstoffatom oder eine $C_{1-6}$-Alkylgruppe ist;

n den Wert 2 oder 3 hat;

und die physiologisch annehmbaren Salze und Solvate davon.

2. Verbindungen nach Anspruch 1, dadurch **gekennzeichnet,** daß $R^1$ für eine $C_{1-4}$-Alkyl-, $C_{3-4}$-Alkinyl-, $C_{5-6}$-Cycloalkyl-, $C_{5-6}$-Cycloalkylmethyl-, Phenyl-$C_{1-2}$-alkyl-, Phenylmethoxymethyl- oder N,N-Di-$C_{1-3}$-alkylcarboxamidogruppe steht.

3. Verbindungen nach Anspruch 1 oder 2, dadurch **gekennzeichnet,** daß $R^2$, $R^3$ und $R^4$ jeweils unabhängig voneinander für ein Wasserstoffatom oder eine $C_{1-3}$-Alkylgruppe stehen.

4. Verbindungen nach Anspruch 1, dadurch **gekennzeichnet,** daß $R^1$ für ein Wasserstoffatom oder eine $C_{1-4}$-Alkyl-, $C_{3-4}$-Alkenyl-, $C_{3-4}$-Alkinyl-, $C_{5-6}$-Cycloalkyl-, $C_{5-6}$-Cycloalkylmethyl-, Phenyl-$C_{1-2}$-alkyl-, Phenylmethoxymethyl-, N,N-Di-$C_{1-3}$-alkylcarboxamido- oder $C_{1-3}$-Alkylsulfonylgruppe steht; $R^2$ für ein Wasserstoffatom steht; und $R^3$ und $R^4$ jeweils für ein Wasserstoffatom oder eine $C_{1-3}$-Alkylgruppe

stehen.

**5.** Verbindungen nach Anspruch 1, dadurch **gekennzeichnet,** daß $R^1$ für eine Methyl-, n-Propyl-, Prop-2-inyl-, Cyclopentyl-, Cyclopentylmethyl-, Benzyl- oder N,N-Dimethylcarboxamidogruppe steht; $R^2$ und $R^3$ jeweils für ein Wasserstoffatom stehen; und $R^4$ für eine Methylgruppe steht.

**6.** Verbindungen nach Anspruch 4 oder 5, dadurch **gekennzeichnet,** daß n den Wert 2 hat.

**7.** 2,3,4,5-Tetrahydro-5-methyl-2-[(5-methyl-1H-imidazol-4-yl)methyl]-1H-pyrido[4,3-b]indol-1-on und die physiologisch annehmbaren Salze und Solvate davon.

**8.** 2,3,4,5-Tetrahydro-5-(phenylmethyl)-2-[(5-methyl-1H-imidazol-4-yl)methyl]-1H-pyrido[4,3-b]indol-1-on;
5-Cyclopentyl-2,3,4,5-tetrahydro-2-[(5-methyl-1H-imidazol-4-yl)methyl]-1H-pyrido[4,3-b]indol-1-on;
2,3,4,5-Tetrahydro-2-[(5-methyl-1H-imidazol-4-yl)methyl]-5-propyl-1H-pyrido[4,3-b]indol-1-on;
5-(Cyclopentylmethyl)-2,3,4,5-tetrahydro-2-[(5-methyl-1H-imidazol-4-yl)methyl]-1H-pyrido[4,3-b]-indol-1-on;
3,4,5,6-Tetrahydro-6-methyl-2-[(5-methyl-1H-imidazol-4-yl)methyl]azepino[4,3-b]indol-1(2H)on;
2,3,4,5-Tetrahydro-N,N-dimethyl-2-[(5-methyl-1H-imidazol-4-yl)methyl]-1-oxo-5H-pyrido[4,3-b]indol-5-carboxamid;
2,3,4,5-Tetrahydro-2-[(5-methyl-1H-imidazol-4-yl)methyl]-5-(2-propinyl)-1H-pyrido[4,3-b]indol-1-on;
und die physiologisch annehmbaren Salze und Solvate davon.

**9.** Verbindungen nach einem der Ansprüche 1 bis 8, dadurch **gekennzeichnet,** daß die Verbindung der Formel (I) in der Form eines Hydrochlorid-, Hydrobromid-, Sulfat-, Alkylsulfonat-, Arylsulfonat-, Phosphat-, Acetat-, Citrat-, Succinat-, Tartrat-, Fumarat- oder Maleatsalzes vorliegt.

**10.** Verbindung nach Anspruch 7, dadurch **gekennzeichnet,** daß sie in der Form eines Hydrochloridsalzes vorliegt.

**11.** Verbindung nach Anspruch 7, dadurch **gekennzeichnet,** daß sie in der Form eines Maleatsalzes vorliegt.

**12.** Verfahren zur Herstellung von Verbindungen der allgemeinen Formel (I) nach einem der Ansprüche 1 bis 11 oder eines physiologisch annehmbaren Salzes oder Solvats davon, dadurch **gekennzeichnet,** daß man

(A) eine Verbindung der Formel (II):

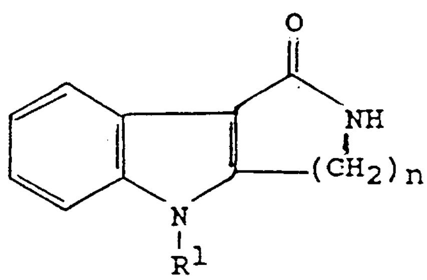

(II)

mit einer Verbindung der Formel (III):

$LCH_2$-Im (III)

oder einem geschützten Derivat davon, worin L ein Austrittsatom oder eine Austrittsgruppe darstellt, alkyliert und anschließend erforderlichenfalls irgendwelche vorhandenen Schutzgruppen entfernt; oder daß man

(B) zur Herstellung einer Verbindung der Formel (I), bei der n den Wert 2 hat, eine Verbindung der Formel (IV):

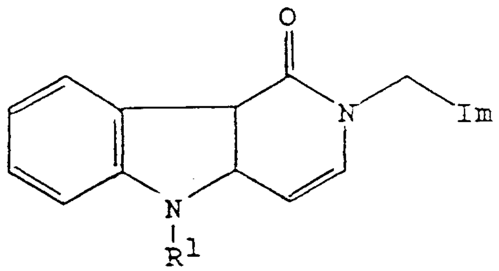

(IV)

oder ein geschütztes Derivat davon hydriert und anschließend erforderlichenfalls irgendwelche vorhandenen Schutzgruppen entfernt; oder daß man
(C) eine Verbindung der Formel (V):

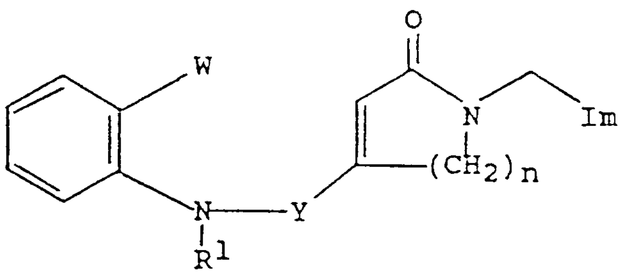

(V)

worin W für ein Wasserstoffatom steht und Y die Gruppe NH ist oder W für ein Halogenatom steht und Y eine Bindung darstellt, oder ein Salz oder ein geschütztes Derivat davon cyclisiert und anschließend erforderlichenfalls irgendwelche vorhandenen Schutzgruppen entfernt; oder daß man
(D) zur Herstellung einer Verbindung der Formel (I), bei der $R^3$ für ein Wasserstoffatom steht, eine Verbindung der Formel (VI):

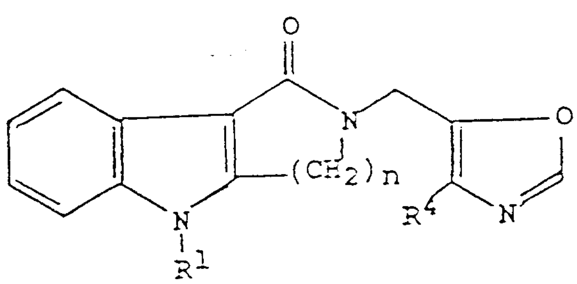

(VI)

oder ein geschütztes Derivat davon mit Formamid umsetzt und anschließend erforderlichenfalls irgendwelche vorhandenen Schutzgruppen entfernt; oder daß man
(E) eine Verbindung der Formel (VII):

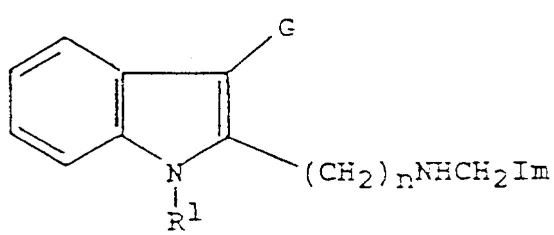

(VII)

worin G ein Wasserstoffatom darstellt, oder ein geschütztes Derivat davon mit Phosgen in Gegenwart einer Lewis-Säure umsetzt; oder

worin G ein Brom- oder Iodatom darstellt, oder ein geschütztes Derivat davon mit Kohlenmonoxid in Gegenwart eines Palladium(II)-salzes umsetzt und anschließend erforderlichenfalls irgendwelche vorhandenen Schutzgruppen entfernt; oder daß man
(F) eine Verbindung der allgemeinen Formel (I) in eine andere Verbindung der Formel (I) unter Anwendung von herkömmlichen Techniken umwandelt; oder daß man
(G) eine Schutzgruppe bzw. Schutzgruppen von einer geschützten Form einer Verbindung der Formel (I) entfernt;
und wenn die Verbindung der Formel (I) als Gemisch von Enantiomeren erhalten wird, gegebenenfalls das Gemisch aufspaltet, um das gewünschte Enantiomere zu erhalten;
und/oder wenn die Verbindung der Formel (I) in der Form einer freien Base vorliegt, die freie Base gegebenenfalls in ein Salz umwandelt.

**13.** Pharmazeutisches Präparat, dadurch **gekennzeichnet,** daß es mindestens eine Verbindung der Formel (I) nach Anspruch 1 oder ein physiologisch annehmbares Salz oder Solvat davon zusammen mit mindestens einem physiologisch annehmbaren Träger oder Corrigens enthält.

**14.** Pharmazeutisches Präparat nach Anspruch 13, dadurch **gekennzeichnet,** daß es in einer Form für die orale oder parenterale Verabreichung vorliegt.

**15.** Pharmazeutisches Präparat nach Anspruch 13 oder 14, dadurch **gekennzeichnet,** daß der Wirkstoff 2,3,4,5-Tetrahydro-5-methyl-2-[(5-methyl-1H-imidazol-4-yl)-methyl]-1H-pyrido[4,3-b]indol-1-on oder ein physiologisch annehmbares Salz oder Solvat davon ist.

**16.** Verwendung einer Verbindung der allgemeinen Formel (I) nach einem der Ansprüche 1 bis 11 oder eines physiologisch annehmbaren Salzes oder Solvats davon zur Herstellung eines Arzneimittels zur Behandlung eines Zustands, der durch eine Störung der "neuronalen" 5-HT-Funktion bewirkt worden ist.

**17.** Verwendung einer Verbindung der allgemeinen Formel (I) nach einem der Ansprüche 1 bis 11 oder eines physiologisch annehmbaren Salzes oder Solvats davon zur Herstellung eines Arzneimittels zur Behandlung von Nausea und Erbrechen und/oder zur Beschleunigung der Magenentleerung.

**18.** Verwendung einer Verbindung der allgemeinen Formel (I) nach einem der Ansprüche 1 bis 11 oder eines physiologisch annehmbaren Salzes oder Solvats davon zur Herstellung eines Arzneimittels zur Behandlung des Reizdarmsyndroms.

**19.** Verwendung einer Verbindung der allgemeinen Formel (I) nach einem der Ansprüche 1 bis 11 oder eines physiologisch annehmbaren Salzes oder Solvats davon zur Herstellung eines Arzneimittels zur Behandlung von Dyspepsie.

**20.** Verwendung einer Verbindung der allgemeinen Formel (I) nach einem der Ansprüche 1 bis 11 oder eines physiologisch annehmbaren Salzes oder Solvats davon zur Herstellung eines Arzneimittels zur Behandlung von Rückfluß-Ösophagitis.

**Patentansprüche für folgende Vertragsstaaten : GR, ES**

**1.** Verfahren zur Herstellung von Verbindungen der allgemeinen Formel (I):

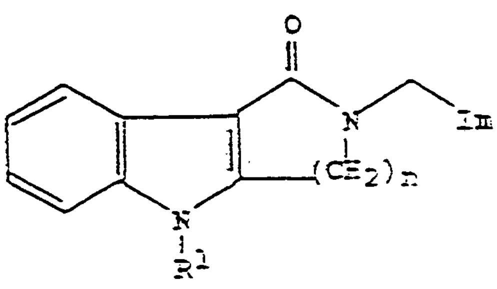

(I)

worin Im für eine Imidazolylgruppe der Formel:

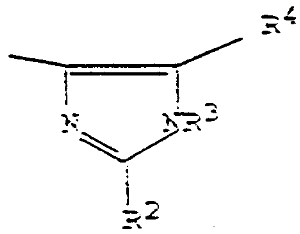

oder

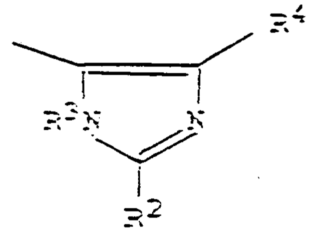

steht;

und $R^1$ für ein Wasserstoffatom oder eine Gruppe, ausgewählt aus $C_{1-6}$-Alkyl, $C_{3-6}$-Alkenyl, $C_{3-10}$-Alkinyl, $c_{3-7}$-Cycloalkyl, $C_{3-7}$-Cyclolalkyl-$C_{1-4}$-alkyl, Phenyl, Phenyl-$C_{1-3}$-alkyl, Phenylmethoxymethyl, Phenoxyethyl, Phenoxymethyl, -$CO_2R^5$, -$COR^5$, -$CONR^5R^6$ oder -$SO_2R^5$, steht (wobei $R^5$ und $R^6$, die gleich oder verschieden sein können, jeweils für ein Wasserstoffatom, eine $C_{1-6}$-Alkyl- oder $C_{3-7}$-Cycloalkylgruppe oder eine Phenyl- oder eine Phenyl-$C_{1-4}$-alkylgruppe stehen, wobei die Phenylgruppe gegebenenfalls durch ein oder mehrere $C_{1-4}$-Alkyl-, $C_{1-4}$-Alkoxy- oder Hydroxygruppen oder Halogenatome substituiert ist, mit der Maßgabe, daß $R^5$ kein Wasserstoffatom ist, wenn $R^1$ für eine Gruppe -$CO_2R^5$ oder -$SO_2R^5$ steht);

eine der Gruppen $R^2$, $R^3$ und $R^4$ ein Wasserstoffatom oder eine $C_{1-6}$-Alkyl-, $C_{3-7}$-Cycloalkyl-, $C_{3-6}$-Alkenyl-, Phenyl- oder Phenyl-$C_{1-3}$-alkylgruppe ist und jede der zwei anderen Gruppen, die gleich oder verschieden sein können, ein Wasserstoffatom oder eine $C_{1-6}$-Alkylgruppe ist;

n den Wert 2 oder 3 hat;

und der physiologisch annehmbaren Salze und Solvate, dadurch **gekennzeichnet,** daß man

(A) eine Verbindung der Formel (II):

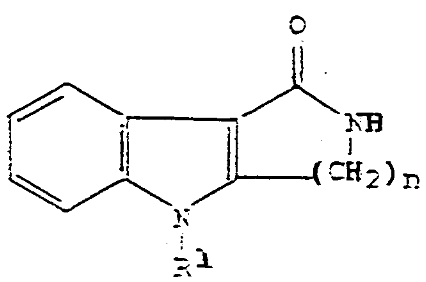

(II)

mit einer Verbindung der Formel (III):

$LCH_2$-Im (III)

oder einem geschützten Derivat davon, worin L ein Austrittsatom oder eine Austrittsgruppe darstellt, alkyliert und anschließend erforderlichenfalls irgendwelche vorhandenen Schutzgruppen entfernt; oder daß man

(B) zur Herstellung einer Verbindung der Formel (I), bei der n den Wert 2 hat, eine Verbindung der Formel (IV):

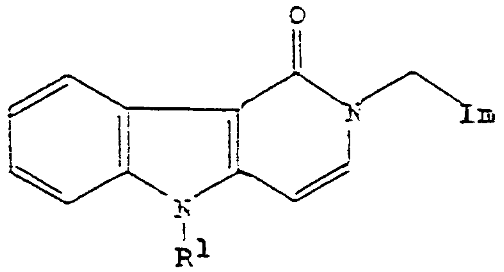

(IV)

oder ein geschütztes Derivat davon hydriert und anschließend erforderlichenfalls irgendwelche vorhandenen Schutzgruppen entfernt; oder daß man
(C) eine Verbindung der Formel (V):

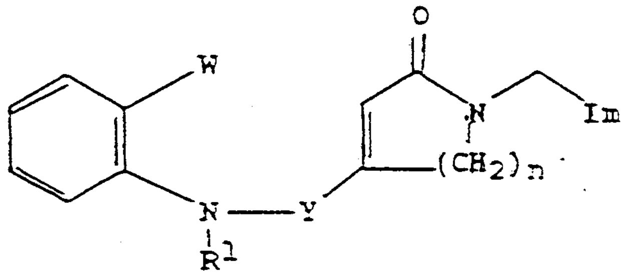

(V)

worin W für ein Wasserstoffatom steht und Y die Gruppe NH ist oder W für ein Halogenatom steht und Y eine Bindung darstellt, oder ein Salz oder ein geschütztes Derivat davon cyclisiert und anschließend erforderlichenfalls irgendwelche vorhandenen Schutzgruppen entfernt; oder daß man
(D) zur Herstellung einer Verbindung der Formel (I), bei der $R^3$ für ein Wasserstoffatom steht, eine Verbindung der Formel (VI):

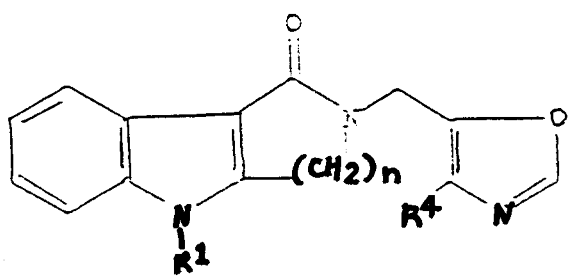

(VI)

oder ein geschütztes Derivat davon mit Formamid umsetzt und anschließend erforderlichenfalls irgendwelche vorhandenen Schutzgruppen entfernt; oder daß man
(E) eine Verbindung der Formel (VII):

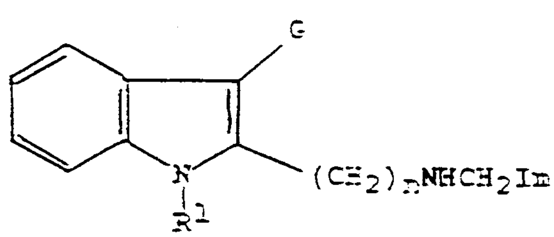

(VII)

worin G ein Wasserstoffatom darstellt, oder ein geschütztes Derivat davon mit Phosgen in Gegenwart einer Lewis-Säure umsetzt; oder

worin G ein Brom- oder Iodatom darstellt, oder ein geschütztes Derivat davon mit Kohlenmonoxid in Gegenwart eines Palladium(II)-salzes umsetzt und anschließend erforderlichenfalls irgendwelche vorhandenen Schutzgruppen entfernt; oder daß man

(F) eine Verbindung der allgemeinen Formel (I) in eine andere Verbindung der Formel (I) unter Anwendung von herkömmlichen Techniken umwandelt; oder daß man

(G) eine Schutzgruppe bzw. Schutzgruppen von einer geschützten Form einer Verbindung der Formel (I) entfernt;

und wenn die Verbindung der Formel (I) als Gemisch von Enantiomeren erhalten wird, gegebenenfalls das Gemisch aufspaltet, um das gewünschte Enantiomere zu erhalten;

und/oder wenn die Verbindung der Formel (I) in der Form einer freien Base vorliegt, die freie Base gegebenenfalls in ein Salz umwandelt.

**2.** Verfahren nach Anspruch 1 zur Herstellung von Verbindungen, bei denen $R^1$ für eine $C_{1-4}$-Alkyl-, $C_{3-4}$-Alkinyl-, $C_{5-6}$-Cycloalkyl-, $C_{5-6}$-Cycloalkylmethyl-, Phenyl-$C_{1-2}$-alkyl-, Phenylmethoxymethyl- oder N,N-Di-$C_{1-3}$-alkylcarboxamidogruppe steht.

**3.** Verfahren nach Anspruch 1 oder 2 zur Herstellung von Verbindungen, bei denen $R^2$, $R^3$ und $R^4$ jeweils unabhängig voneinander für ein Wasserstoffatom oder eine $C_{1-3}$-Alkylgruppe stehen.

**4.** Verfahren nach Anspruch 1 zur Herstellung von Verbindungen, bei denen $R^1$ für ein Wasserstoffatom oder eine $C_{1-4}$-Alkyl-, $C_{3-4}$-Alkenyl-, $C_{3-4}$-Alkinyl-, $C_{5-6}$-Cycloalkyl-, $C_{5-6}$-Cycloalkylmethyl-, Phenyl-$C_{1-2}$-alkyl-, Phenylmethoxymethyl-, N,N-Di-$C_{1-3}$-alkylcarboxamido- oder $C_{1-3}$-Alkylsulfonylgruppe steht; $R^2$ für ein Wasserstoffatom steht; und $R^3$ und $R^4$ jeweils für ein Wasserstoffatom oder eine $C_{1-3}$-Alkylgruppe stehen.

**5.** Verfahren nach Anspruch 1 zur Herstellung von Verbindungen, bei denen $R^1$ für eine Methyl-, n-Propyl-, Prop-2-inyl-, Cyclopentyl-, Cyclopentylmethyl-, Benzyl- oder N,N-Dimethylcarboxamidogruppe steht; $R^2$ und $R^3$ jeweils für ein Wasserstoffatom stehen; und $R^4$ für eine Methylgruppe steht.

**6.** Verfahren nach Anspruch 4 oder 5 zur Herstellung von Verbindungen, bei denen n den Wert 2 hat.

**7.** Verfahren nach Anspruch 1 zur Herstellung der Verbindung 2,3,4,5-Tetrahydro-5-methyl-2-[(5-methyl-1H-imidazol-4-yl)methyl]-1H-pyrido[4,3-b]indol-1-on und der physiologisch annehmbaren Salze und Solvate davon.

**8.** Verfahren nach Anspruch 1 zur Herstellung einer Verbindung, ausgewählt aus:

2,3,4,5-Tetrahydro-5-(phenylmethyl)-2-[(5-methyl-1H-imidazol-4-yl)methyl]-1H-pyrido[4,3-b]indol-1-on;

5-Cyclopentyl-2,3,4,5-tetrahydro-2-[(5-methyl-1H-imidazol-4-yl)methyl]-1H-pyrido[4,3-b]indol-1-on;

2,3,4,5-Tetrahydro-2-[(5-methyl-1H-imidazol-4-yl)-methyl]-5-propyl-1H-pyrido[4,3-b]indol-1-on;

5-(Cyclopentylmethyl)-2,3,4,5-tetrahydro-2-[(5-methyl-1H-imidazol-4-yl)methyl]-1H-pyrido[4,3-b]-indol-1-on;

3,4,5,6-Tetrahydro-6-methyl-2-[(5-methyl-1H-imidazol-4-yl)methyl]azepino[4,3-b]indol-1(2H)on;

2,3,4,5-Tetrahydro-N,N-dimethyl-2-[(5-methyl-1H-imidazol-4-yl)methyl]-1-oxo-5H-pyrido[4,3-b]indol-5-carboxamid;

2,3,4,5-Tetrahydro-2-[(5-methyl-1H-imidazol-4-yl)methyl]-5-(2-propinyl)-1H-pyrido[4,3-b]indol-1-on;

und der physiologisch annehmbaren Salze und Solvate davon.

**9.** Verfahren nach einem der Ansprüche 1 bis 8 zur Herstellung einer Verbindung der Formel (I) in Form eines Hydrochlorid-, Hydrobromid-, Sulfat-, Alkylsulfonat-, Arylsulfonat-, Phosphat-, Acetat-, Citrat-, Succinat-, Tartrat-, Fumarat- oder Maleatsalzes.

**10.** Verfahren nach Anspruch 7 zur Herstellung der Verbindung in Form eines Hydrochloridsalzes.

**11.** Verfahren nach Anspruch 7 zur Herstellung der Verbindung in Form eines Maleatsalzes.

**12.** Verwendung einer Verbindung der allgemeinen Formel (I) nach einem der Ansprüche 1 bis 11 oder eines physiologisch annehmbaren Salzes oder Solvats davon zur Herstellung eines Arzneimittels zur

Behandlung eines Zustands, der durch eine Störung der "neuronalen" 5-HT-Funktion bewirkt worden ist.

**13.** Verwendung einer Verbindung der allgemeinen Formel (I) nach einem der Ansprüche 1 bis 11 oder eines physiologisch annehmbaren Salzes oder Solvats davon zur Herstellung eines Arzneimittels zur Behandlung von Nausea und Erbrechen und/oder zur Beschleunigung der Magenentleerung.

**14.** Verwendung einer Verbindung der allgemeinen Formel (I) nach einem der Ansprüche 1 bis 11 oder eines physiologisch annehmbaren Salzes oder Solvats davon zur Herstellung eines Arzneimittels zur Behandlung des Reizdarmsyndroms.

**15.** Verwendung einer Verbindung der allgemeinen Formel (I) nach einem der Ansprüche 1 bis 11 oder eines physiologisch annehmbaren Salzes oder Solvats davon zur Herstellung eines Arzneimittels zur Behandlung von Dyspepsie.

**16.** Verwendung einer Verbindung der allgemeinen Formel (I) nach einem der Ansprüche 1 bis 11 oder eines physiologisch annehmbaren Salzes oder Solvats davon zur Herstellung eines Arzneimittels zur Behandlung von Rückfluß-Ösophagitis.

## Revendications

**Revendications pour les Etats contractants suivants : AT, BE, CH, DE, FR, GB, IT, LI, LU, NL, SE**

**1.** Composés de formule générale (I)

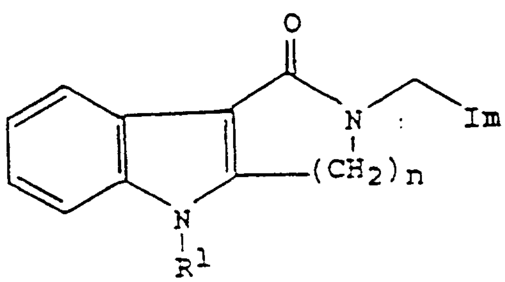

(I)

dans laquelle Im représente un groupe imidazolyle de formule:

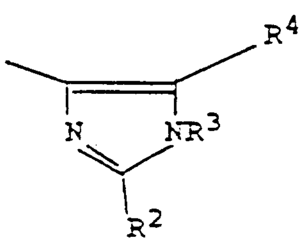

ou

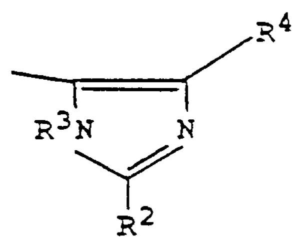

et $R^1$ représente un atome d'hydrogène ou un groupe choisi parmi un groupe alkyle en $C_{1-6}$, alcényle en $C_{3-6}$, alcynyle en $C_{3-10}$, cycloalkyle en $C_{3-7}$, cycloalkyl($C_{3-7}$)-alkyle($C_{1-4}$), phényle, phénylalkyle($C_{1-3}$), phénylméthoxyméthyle, phénoxyéthyle, phénoxyméthyle, $-CO_2R^5$, $-COR^5$, $-CONR^5R^6$ ou $-SO_2R^5$ (dans lesquels $R^5$ et $R^6$, qui peuvent être identiques ou différents, représentent chacun un atome d'hydrogène, un groupe alkyle en $C_{1-6}$ ou cycloalkyle en $C_{3-7}$ ou un groupe phényle ou phénylalkyle($C_{1-4}$), dans lesquels le radical phényle est éventuellement substitué par un ou plusieurs groupes alkyle en $C_{1-4}$, alcoxy en $C_{1-4}$ ou hydroxy ou atomes d'halogène, étant entendu que $R^5$ ne représente pas un atome d'hydrogène lorsque $R^1$ représente un groupe $-CO_2R^5$ ou $-SO_2R^5$);

l'un des groupes représentés par $R^2$, $R^3$ et $R^4$ est un atome d'hydrogène ou un groupe alkyle en $C_{1-6}$, cycloalkyle en $C_{3-7}$, alcényle en $C_{3-6}$, phényle ou phénylalkyle($C_{1-3}$), et chacun des deux

autres groupes, qui peuvent être identiques ou différents, représente un atome d'hydrogène ou un groupe alkyle en $C_{1-6}$;

n représente 2 ou 3;

et sels physiologiquement acceptables et produits de solvatation de ceux-ci.

**2.** Composés selon la revendication 1, dans lesquels $R^1$ représente un groupe alkyle en $C_{1-4}$, alcynyle en $C_{3-4}$, cycloalkyle en $C_{5-6}$, cycloalkyl($C_{5-6}$)méthyle, phénylalkyle($C_{1-2}$), phénylméthoxyméthyle ou N,N-dialkyl($C_{1-3}$)carboxamido.

**3.** Composés selon la revendication 1 ou 2, dans lesquels $R^2$, $R^3$ et $R^4$ représentent chacun indépendamment un atome d'hydrogène ou un groupe alkyle en $C_{1-3}$.

**4.** Composés selon la revendication 1, dans lesquels $R^1$ représente un atome d'hydrogène ou un groupe alkyle en $C_{1-4}$, alcényle en $C_{3-4}$, alcynyle en $C_{3-4}$, cycloalkyle en $C_{5-6}$, cycloalkyl($C_{5-6}$)méthyle, phénylalkyle($C_{1-2}$), phénylméthoxyméthyle, N,N-dialkyl($C_{1-3}$)carboxamido ou alkyl($C_{1-3}$)-sulfonyle; $R^2$ représente un atome d'hydrogène; et $R^3$ et $R^4$ représentent chacun un atome d'hydrogène ou un groupe alkyle en $C_{1-3}$.

**5.** Composés selon la revendication 1, dans lesquels $R^1$ représente le groupe méthyle, n-propyle, prop-2-ynyle, cyclopentyle, cyclopentylméthyle, benzyle ou N,N-diméthylcarboxamido; $R^2$ et $R^3$ représentent chacun un atome d'hydrogène; et $R^4$ représente le groupe méthyle.

**6.** Composés selon la revendication 4 ou 5, dans lesquels n représente 2.

**7.** 2,3,4,5-tétrahydro-5-méthyl-2-[(5-méthyl-1H-imidazol-4-yl)méthyl]-1H-pyrido[4,3-b]indol-1-one, et sels physiologiquement acceptables et produits de solvatation de ce composé.

**8.** 2,3,4,5-tétrahydro-5-(phénylméthyl)-2-[(5-méthyl-1H-imidazol-4-yl)méthyl]-1H-pyrido[4,3-b]indol-1-one,
5-cyclopentyl-2,3,4,5-tétrahydro-2-[(5-méthyl-1H-imidazol-4-yl)méthyl]-1H-pyrido[4,3-b]indol-1-one,
2,3,4,5-tétrahydro-2-[(5-méthyl-1H-imidazol-4-yl)méthyl]-5-propyl]-1H-pyrido[4,3-b]indol-1-one,
5-(cyclopentylméthyl)-2,3,4,5-tétrahydro-2-[(5-méthyl-1H-imidazol-4-yl)méthyl]-1H-pyrido[4,3-b]indol-1-one,
3,4,5,6-tétrahydro-6-méthyl-2-[(5-méthyl-1H-imidazol-4-yl)méthyl]-azépino[4,3-b]indol-1(2H)-one,
2,3,4,5-tétrahydro-N,N-diméthyl-2-[(5-méthyl-1H-imidazol-4-yl)méthyl]-1-oxo-5H-pyrido[4,3-b]indole-5-carboxamide,
2,3,4,5-tétrahydro-2-[(5-méthyl-1H-imidazol-4-yl)méthyl]-5-(2-propynyl)-1H-pyrido[4,3-b]indol-1-one,
et sels physiologiquement acceptables et produits de solvatation de ces composés.

**9.** Composés selon l'une quelconque des revendications 1 à 8, dans lesquels le composé de composé formule (I) est sous la forme d'un chlorhydrate, bromhydrate, sulfate, alkylsulfonate, arylsulfonate, phosphate, acétate, citrate, succinate, tartrate, fumarate ou maléate.

**10.** Composé selon la revendication 7, sous la forme de chlorhydrate.

**11.** Composé selon la revendication 7, sous la forme de maléate.

**12.** Procédé pour la préparation de composés de formule générale (I) telle que définie dans l'une quelconque des revendications 1 à 11, ou de sels physiologiquement acceptables ou produits de solvatation de ceux-ci, comprenant:

(A) l'alkylation d'un composé de formule (II)

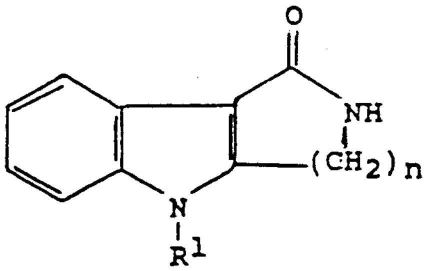

(II)

par un composé de formule (III)

$LCH_2$-Im (III)

ou un dérivé protégé de celui-ci, dans lequel L représente un atome ou groupe partant, suivie si nécessaire de l'élimination de tous groupes protecteurs présents; ou

(B) pour la préparation d'un composé de formule (I) dans lequel n est 2, l'hydrogénation d'un composé de formule (IV)

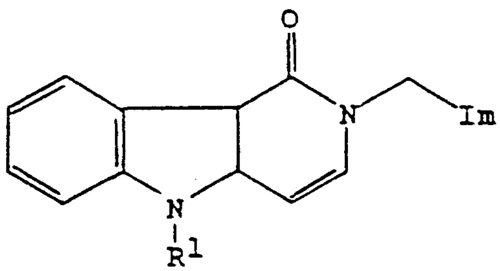

(IV)

ou d'un dérivé protégé de celui-ci, suivie si nécessaire de l'élimination de tous groupes protecteurs présents; ou

(C) la cyclisation d'un composé de formule (V)

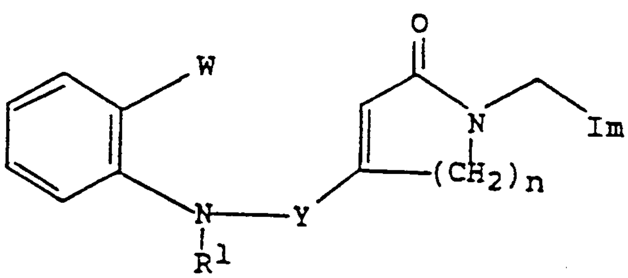

(V)

dans laquelle W représente un atome d'hydrogène et Y représente le groupe NH, ou W représente un atome d'halogène et Y représente une liaison, ou d'un sel ou dérivé protégé de celui-ci, suivie si nécessaire de l'élimination de tous groupes protecteurs présents; ou

(D) pour la préparation d'un composé de formule (I) dans lequel $R^3$ représente un atome d'hydrogène, la mise en réaction d'un composé de formule (VI)

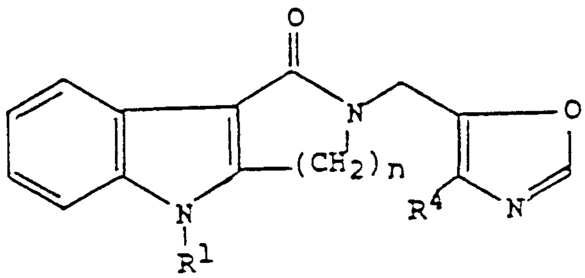

(VI)

ou d'un dérivé protégé de celui-ci, avec du formamide, suivie si nécessaire de l'élimination de tous groupes protecteurs présents; ou

(E) la mise en réaction d'un composé de formule (VII)

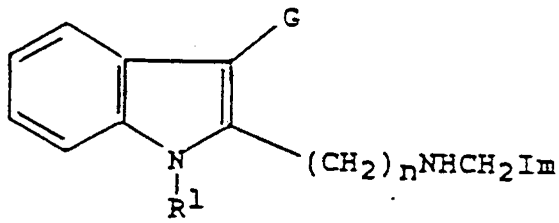

(VII)

dans laquelle G représente un atome d'hydrogène, ou d'un dérivé protégé de celui-ci, avec du phosgène en présence d'un acide de Lewis; ou

lorsque G représente un atome de brome ou d'iode, ou un dérivé protégé de celui-ci, avec du monoxyde de carbone en présence d'un sel de palladium-(II); suivie si nécessaire de l'élimination de tous groupes protecteurs présents; ou

(F) la conversion d'un composé de formule générale (I) en un autre composé de formule (I), à l'aide de techniques classiques; ou

(G) l'élimination de groupe(s) protecteur(s) d'une forme protégée d'un composé de formule (I);

et, lorsque le composé de formule (I) est obtenu sous forme d'un mélange d'énantiomères, éventuellement la résolution du mélange pour l'obtention de l'énantiomère recherché;

et/ou lorsque le composé de formule (I) est sous la forme d'une base libre, éventuellement la conversion de la base libre en un sel.

**13.** Composition pharmaceutique comprenant au moins un composé de formule générale (I) telle que définie dans la revendication 1, ou un sel physiologiquement acceptable ou produit de solvatation de celui-ci, conjointement avec au moins un excipient ou véhicule physiologiquement acceptable.

**14.** Composition pharmaceutique selon la revendication 13, sous une forme adaptée à l'administration orale ou parentérale.

**15.** Composition pharmaceutique selon la revendication 13 ou 14, dans laquelle le composant actif est la 2,3,4,5-tétrahydro-5-méthyl-2-[(5-méthyl-1H-imidazol-4-yl)méthyl]-1H-pyrido[4,3-b]indol-1-one ou un sel physiologiquement acceptable ou produit de solvatation de celle-ci.

**16.** Utilisation d'un composé de formule générale (I) telle que définie dans l'une quelconque des revendications 1 à 11, ou d'un sel physiologiquement acceptable ou produit de solvatation de celui-ci, pour la fabrication d'un médicament pour le traitement d'un état provoqué par un trouble de la fonction "neuronale" de la 5-HT.

**17.** Utilisation d'un composé de formule générale (I) telle que définie dans l'une quelconque des revendications 1 à 11, ou d'un sel physiologiquement acceptable ou produit de solvatation de celui-ci, pour la fabrication d'un médicament pour le traitement de la nausée et de vomissements et/ou pour la facilitation de la vidange gastrique.

**18.** Utilisation d'un composé de formule générale (I) telle que définie dans l'une quelconque des revendications 1 à 11, ou d'un sel physiologiquement acceptable ou produit de solvatation de celui-ci, pour la fabrication d'un médicament pour le traitement du syndrome de l'intestin irritable.

**19.** Utilisation d'un composé de formule générale (I) telle que définie dans l'une quelconque des revendications 1 à 11, ou d'un sel physiologiquement acceptable ou produit de solvatation de celui-ci, pour la fabrication d'un médicament pour le traitement de la dyspepsie.

**20.** Utilisation d'un composé de formule générale (I) telle que définie dans l'une quelconque des revendications 1 à 11, ou d'un sel physiologiquement acceptable ou produit de solvatation de celui-ci, pour la fabrication d'un médicament pour le traitement du reflux oesophagien.

**Revendications pour les Etats contractants suivants : GR, ES**

**1.** Procédé pour la préparation de composés de formule générale (I)

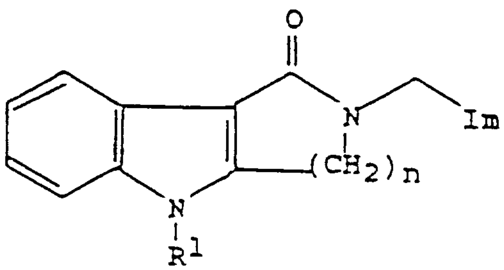

(I)

dans laquelle Im représente un groupe imidazolyle de formule:

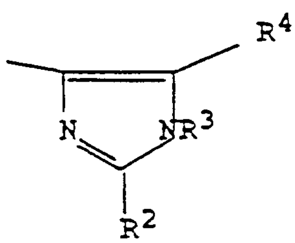

ou

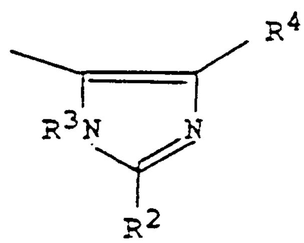

et $R^1$ représente un atome d'hydrogène ou un groupe choisi parmi un groupe alkyle en $C_{1-6}$, alcényle en $C_{3-6}$, alcynyle en $C_{3-10}$, cycloalkyle en $C_{3-7}$, cycloalkyl($C_{3-7}$)-alkyle($C_{1-4}$), phényle, phénylalkyle($C_{1-3}$), phénylméthoxyméthyle, phénoxyéthyle, phénoxyméthyle, $-CO_2R^5$, $-COR^5$, $-CONR^5R^6$ ou $-SO_2R^5$ (dans lesquels $R^5$ et $R^6$, qui peuvent être identiques ou différents, représentent chacun un atome d'hydrogène, un groupe alkyle en $C_{1-6}$ ou cycloalkyle en $C_{3-7}$ ou un groupe phényle ou phénylalkyle($C_{1-4}$), dans lesquels le radical phényle est éventuellement substitué par un ou plusieurs groupes alkyle en $C_{1-4}$, alcoxy en $C_{1-4}$ ou hydroxy ou atomes d'halogène, étant entendu que $R^5$ ne représente pas un atome d'hydrogène lorsque $R^1$ représente un groupe $-CO_2R^5$ ou $-SO_2R^5$);

l'un des groupes représentés par $R^2$, $R^3$ et $R^4$ est un atome d'hydrogène ou un groupe alkyle en $C_{1-6}$, cycloalkyle en $C_{3-7}$, alcényle en $C_{3-6}$, phényle ou phénylalkyle($C_{1-3}$), et chacun des deux autres groupes, qui peuvent être identiques ou différents, représente un atome d'hydrogène ou un groupe alkyle en $C_{1-6}$;

n représente 2 ou 3;

et de leurs sels physiologiquement acceptables et produits de solvatation,
comprenant:

(A) l'alkylation d'un composé de formule (II)

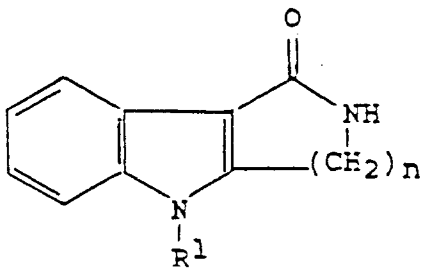

(II)

par un composé de formule (III)

$LCH_2$-Im (III)

ou un dérivé protégé de celui-ci, dans lequel L représente un atome ou groupe partant, suivie si nécessaire de l'élimination de tous groupes protecteurs présents; ou

(B) pour la préparation d'un composé de formule (I) dans lequel n est 2, l'hydrogénation d'un composé de formule (IV)

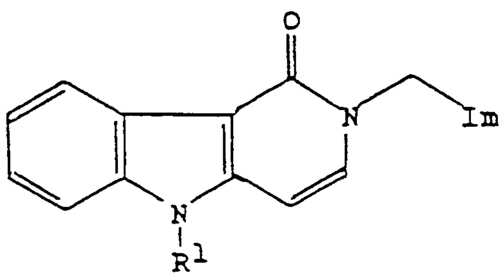

(IV)

ou d'un dérivé protégé de celui-ci, suivie si nécessaire de l'élimination de tous groupes protecteurs présents; ou

(C) la cyclisation d'un composé de formule (V)

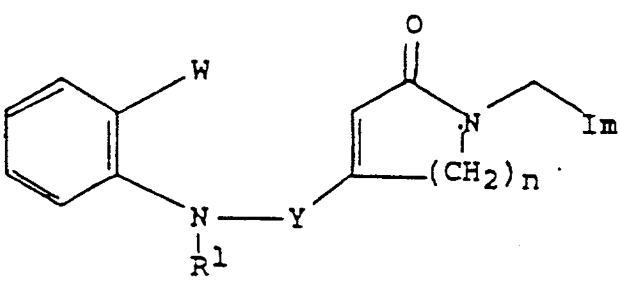

(V)

dans laquelle W représente un atome d'hydrogène et Y représente le groupe NH, ou W représente un atome d'halogène et Y représente une liaison, ou d'un sel ou dérivé protégé de celui-ci, suivie si nécessaire de l'élimination de tous groupes protecteurs présents; ou

(D) pour la préparation d'un composé de formule (I) dans lequel $R^3$ représente un atome d'hydrogène, la mise en réaction d'un composé de formule (VI)

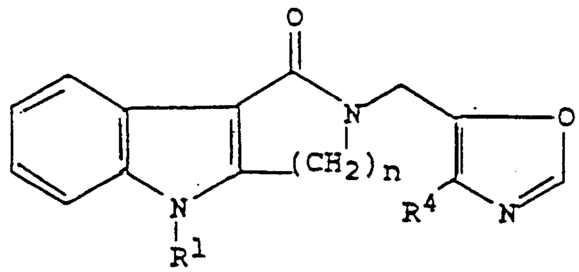

(VI)

ou d'un dérivé protégé de celui-ci, avec du formamide, suivie si nécessaire de l'élimination de tous groupes protecteurs présents; ou

(E) la mise en réaction d'un composé de formule (VII)

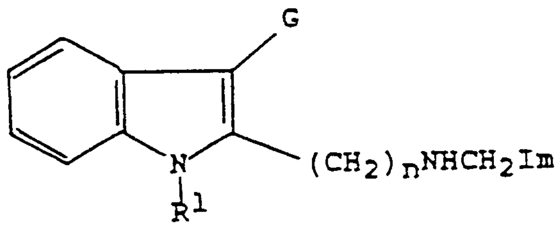

(VII)

dans laquelle G représente un atome d'hydrogène, ou d'un dérivé protégé de celui-ci, avec du phosgène en présence d'un acide de Lewis; ou lorsque G représente un atome de brome ou d'iode, ou un dérivé protégé de celui-ci, avec du monoxyde de carbone en présence d'un sel de palladium-(II); suivie si nécessaire de l'élimination de tous groupes protecteurs présents; ou

(F) la conversion d'un composé de formule générale (I) en un autre composé de formule (I), à l'aide de techniques classiques; ou

(G) l'élimination de groupe(s) protecteur(s) d'une forme protégée d'un composé de formule (I);

et, lorsque le composé de formule (I) est obtenu sous forme d'un mélange d'énantiomères, éventuellement la résolution du mélange pour l'obtention de l'énantiomère recherché;

et/ou lorsque le composé de formule (I) est sous la forme d'une base libre, éventuellement la conversion de la base libre en un sel.

**2.** Procédé selon la revendication 1, pour la préparation de composés dans lesquels $R^1$ représente un groupe alkyle en $C_{1-4}$, alcynyle en $C_{3-4}$, cycloalkyle en $C_{5-6}$, cycloalkyl($C_{5-6}$)méthyle, phénylalkyle-($C_{1-2}$), phénylméthoxyméthyle ou N,N-dialkyl($C_{1-3}$)carboxamido.

**3.** Procédé selon la revendication 1 ou 2, pour la préparation de composés dans lesquels $R^2$, $R^3$ et $R^4$ représentent chacun indépendamment un atome d'hydrogène ou un groupe alkyle en $C_{1-3}$.

**4.** Procédé selon la revendication 1, pour la préparation de composés dans lesquels $R^1$ représente un atome d'hydrogène ou un groupe alkyle en $C_{1-4}$, alcényle en $C_{3-4}$, alcynyle en $C_{3-4}$, cycloalkyle en $C_{5-6}$, cycloalkyl($C_{5-6}$)-méthyle, phénylalkyle($C_{1-2}$), phénylméthoxyméthyle, N,N-dialkyl($C_{1-3}$)-carboxamido ou alkyl($C_{1-3}$)sulfonyle; $R^2$ représente un atome d'hydrogène; et $R^3$ et $R^4$ représentent chacun un atome d'hydrogène ou un groupe alkyle en $C_{1-3}$.

**5.** Procédé selon la revendication 1, pour la préparation de composés dans lesquels $R^1$ représente le groupe méthyle, n-propyle, prop-2-ynyle, cyclopentyle, cyclopentylméthyle, benzyle ou N,N-diméthyl-carboxamido; $R^2$ et $R^3$ représentent chacun un atome d'hydrogène; et $R^4$ représente le groupe méthyle.

**6.** Procédé selon la revendication 4 ou 5, pour la préparation de composes dans lesquels n représente 2.

**7.** Procédé selon la revendication 1, pour la préparation du composé 2,3,4,5-tétrahydro-5-méthyl-2-[(5-méthyl-1H-imidazol-4-yl)méthyl]-1H-pyrido[4,3-b]indol-1-one, et de ses sels physiologiquement acceptables et produits de solvatation.

8. Procédé selon la revendication 1, pour la préparation d'un composé choisi parmi:
la 2,3,4,5-tétrahydro-5-(phénylméthyl)-2-[(5-méthyl-1H-imidazol-4-yl)méthyl]-1H-pyrido[4,3-b]indol-1-one,
la 5-cyclopentyl-2,3,4,5-tétrahydro-2-[(5-méthyl-1H-imidazol-4-yl)méthyl]-1H-pyrido[4,3-b]indol-1-one,
la 2,3,4,5-tétrahydro-2-[(5-méthyl-1H-imidazol-4-yl)méthyl]-5-propyl]-1H-pyrido[4,3-b]indol-1-one,
la 5-(cyclopentylméthyl)-2,3,4,5-tétrahydro-2-[(5-méthyl-1H-imidazol-4-yl)méthyl)-1H-pyrido[4,3-b]indol-1-one,
la 3,4,5,6-tétrahydro-6-méthyl-2-[(5-méthyl-1H-imidazol-4-yl)méthyl]-azépino[4,3-b]indol-1(2H)-one,
le 2,3,4,5-tétrahydro-N,N-diméthyl-2-[(5-méthyl-1H-imidazol-4-yl)méthyl]-1-oxo-5H-pyrido[4,3-b]indole-5-carboxamide,
la 2,3,4,5-tétrahydro-2-[(5-méthyl-1H-imidazol-4-yl)méthyl]-5-(2-propynyl)-1H-pyrido[4,3-b]indol-1-one,
et leurs sels physiologiquement acceptables et produits de solvatation.

9. Procédé selon l'une quelconque des revendications 1 à 8, pour la préparation d'un composé de formule (I) sous la forme d'un chlorhydrate, bromhydrate, sulfate, alkylsulfonate, arylsulfonate, phosphate, acétate, citrate, succinate, tartrate, fumarate ou maléate.

10. Procédé selon la revendication 7, pour la préparation du composé sous la forme de chlorhydrate.

11. Procédé selon la revendication 7, pour la préparation du composé sous la forme de maléate.

12. Utilisation d'un composé de formule générale (I) telle que définie dans l'une quelconque des revendications 1 à 11, ou d'un sel physiologiquement acceptable ou produit de solvatation de celui-ci, pour la fabrication d'un médicament pour le traitement d'un état provoqué par un trouble de la fonction "neuronale" de la 5-HT.

13. Utilisation d'un composé de formule générale (I) telle que définie dans l'une quelconque des revendications 1 à 11, ou d'un sel physiologiquement acceptable ou produit de solvatation de celui-ci, pour la fabrication d'un médicament pour le traitement de la nausée et de vomissements et/ou pour la facilitation de la vidange gastrique.

14. Utilisation d'un composé de formule générale (I) telle que définie dans l'une quelconque des revendications 1 à 11, ou d'un sel physiologiquement acceptable ou produit de solvatation de celui-ci, pour la fabrication d'un médicament pour le traitement du syndrome de l'intestin irritable.

15. Utilisation d'un composé de formule générale (I) telle que définie dans l'une quelconque des revendications 1 à 11, ou d'un sel physiologiquement acceptable ou produit de solvatation de celui-ci, pour la fabrication d'un médicament pour le traitement de la dyspepsie.

16. Utilisation d'un composé de formule générale (I) telle que définie dans l'une quelconque des revendications 1 à 11, ou d'un sel physiologiquement acceptable ou produit de solvatation de celui-ci, pour la fabrication d'un médicament pour le traitement du reflux oesophagien.